# EUROPEAN PATENT SPECIFICATION

(11) **EP 1 697 500 B1**
(45) Date of publication and mention of the grant of the patent: **20.03.2013**
(21) Application number: 04806164.2
(22) Date of filing: 20.12.2004
(51) Int. Cl.: C12N 5/074, C12N 5/10, A61K 35/12, A61K 48/00, G01N 33/00

(54) **STEM CELLS**
STAMMZELLEN
CELLULES SOUCHES

(30) Priority: 19.12.2003 GB 0329449
(43) Date of publication of application: 06.09.2006
(73) Proprietor: Omnicyte Ltd., London EC2M 2TD (GB)
(72) Inventor: GORDON, Myrtle, Binfield, Berkshire RG42 5QH (GB); HABIB, Nagy, Castlebar Hill, London W5 1TA (GB)
(74) Representative: Rigby, Barbara Nicole
(86) International application number: PCT/GB2004/005365
(87) International publication number: WO 2005/059113

(56) References cited:
- WO-A-01/71016
- WO-A-02/064748
- BUCALA R ET AL: "CIRCULATING FIBROCYTES DEFINE A NEW LEUKOCYTE SUBPOPULATION THAT MEDIATES TISSUE REPAIR" MOLECULAR MEDICINE, BLACKWELL SCIENCE, CAMBRIDGE, MA, US, vol. 1, no. 1, November 1994 (1994-11), pages 71-81, XP002051352 ISSN: 1076-1551
- VERFAILLIE C M: "Adult stem cells: Assessing the case for pluripotency" TRENDS IN CELL BIOLOGY, ELSEVIER SCIENCE LTD, XX, vol. 12, no. 11, November 2002 (2002-11), pages 502-508, XP002268623 ISSN: 0962-8924
- DEANS ROBERT J ET AL: "Mesenchymal stem cells: Biology and potential clinical uses" EXPERIMENTAL HEMATOLOGY, NEW YORK, NY, US, vol. 28, no. 8, August 2000 (2000-08), pages 875-884, XP002201188 ISSN: 0301-472X
- JIANG Y ET AL: "PLURIPOTECNY OF MESENCHYMAL STEM CELLS DERUVED FROM ADULT MARROW" NATURE, MACMILLAN JOURNALS LTD. LONDON, GB, vol. 418, no. 6893, 4 July 2002 (2002-07-04), pages 41-49, XP001204372 ISSN: 0028-0836
- JAVAZON ELISABETH H ET AL: "Mesenchymal stem cells: Paradoxes of passaging" EXPERIMENTAL HEMATOLOGY (NEW YORK), vol. 32, no. 5, May 2004 (2004-05), pages 414-425, XP002321064 ISSN: 0301-472X cited in the application

## Description

The present invention relates to methods of producing certain target cells from a new type of stem cells that can be isolated from bone marrow and blood.

Stem cells can produce new cells to repair damage to any tissue in the body and therefore have immense potential for all types of regenerative medicine. Stem cells are present in all body tissues and organs but some, like bone marrow and blood, are more accessible than others, like liver and brain. However, stem cells exist in very small numbers in marrow and blood, and need to be extracted then increased in number ("expanded") before they can be used clinically. Currently, many attempts are being made to accomplish the aim of providing stem cells in sufficient numbers to perform tissue-specific stem cell transplantation.

Efforts have focussed on the bone marrow as a source of stem cells. Evidence to date suggests that the bone marrow contains two types of stem cells - haemopoietic stem cells (HSC) responsible for producing blood, and mesenchymal stem cells (MSC) capable of producing cells belonging to a limited range of body tissues. MSC can not be precisely defined or isolated and show a limited capacity to provide multiple cell types. They form osteoblasts, chondroblasts and adipocytes in response to stimulation in culture but there are many cell types, such as hepatocytes, which they cannot form. Prolonged culture of MSC results in the outgrowth of a subpopulation of cells referred to as multipotent adult progenitor cells (MAPC) which to date appear to have the broadest potential for tissue regeneration. However, the fact that prolonged tissue culture and many cell divisions are required before MAPC emerge means, first, that they may have accumulated genetic damage and second, that it is impossible to be certain that they represent normal cellular components of the bone marrow. They may, in fact, be a tissue culture artefact. These important considerations potentially contra-indicate the clinical use of MAPC. MAPC can form cells with endodermal, ectodermal or mesodermal markers but, significantly, do not produce haemopoietic cells in culture.

Using differentiated cells derived from stem cells to produce their natural protein products has advantages over the use of cells to produce recombinant proteins, particularly because of the capability for appropriate glycosylation and post-translational modification of the protein product.

The present inventors have now identified a new type of stem cell which can be directly isolated from adult bone marrow and blood, e.g. peripheral blood, and have the unique ability to differentiate into ectodermal, mesodermal and endodermal cells. These cells are thus clearly multipotent if not totipotent. Thus the stem cells described herein provide a novel source of cells for tissue transplantation that may be used in an autologous (self-to-self) manner. Further, as is described below, these stem cells do not require prolonged tissue culture.

The stem cells of the present invention are preferably obtained from a sample taken from an adult such as adult bone marrow or peripheral blood from an adult. Thus the cells are preferably adult stem cells. The cells may also be obtained from other samples such as the umbilical cord and the stem cell population of the present invention may thus in one embodiment comprise fetal as well as adult cells. Fetal sources, eg fetal liver or bone marrow, may also be used.

The methods of the invention involve an isolated stem cell population wherein said stem cells are CD34⁺, capable of self regeneration and capable of differentiation into ectodermal, mesodermal and endodermal cells, preferably into haemopoietic cells. Preferably, the stem cells are adult stem cells.

These stem cells are further characterised by their ability to adhere to plastic (e.g. the plastic of standard tissue culture vessels) during culturing. The cells are thus "capable of" adhering to plastic in the culturing methods described herein and without any special further conditions or modifications. Suitable vessels are those manufactured by Corning Incorporated, New York, USA.

These stem cells may be further characterised by the fact that they do not require feeder layers, i.e. cells (typically inactivated by gamma irradiation which supply important metabolites without further growth or division of their own) which support the growth of the stem cells. Thus, preferably, a feeder layer is not used during culturing of the stem cells.

One primary characterising and particularly advantageous feature of these stem cells is their ability to differentiate into a very wide variety of different cell types including ectodermal, mesodermal and endodermal cells. Thus, these stem cells
can differentiate into cell types which are developmentally derived from the three germ layers of the embryo; ectoderm, mesoderm and endoderm; for example haemopoietic and muscle cells which are derived from the mesoderm; nerve or epithial cells from the ectoderm; and glandular epithelium or hepatocytes from the endoderm.

The cell population is 'isolated' in that it is substantially free of other cell types. Preferably, it is substantially free of cell types which express CD33, CD38, HLA/DR, CD19 and CD3. 'Substantially free' should be interpreted to be consistent with the empirical data presented in the examples. Also, the population is substantially free of cells dedicated to a particular lineage and/or cells carrying markers associated therewith. Preferably the population has less than 20%, more preferably less than 10%, e.g. less than 5% of lineage committed cells. It may assist in the isolation of the present stem cell population to combine both negative selection (removal of cells) and positive selection (isolation of cells), in both cases antibody binding may be used. 'Isolated' cells include those which have been directly isolated from a sample as well as cells cultured or derived from such a sample.

Stem cells are thought to be manufactured in the adult bone marrow but are also found in the blood. The present stem cells may be collected from either of these sources according to standard sampling techniques. Blood samples are preferably obtained following stem cell mobilisation with G-CSF to increase the numbers of stem cells in the circulation. For example, 5 µg/kg body weight/day may be administered subcutaneously for 5 days. It is also possible to obtain direct bone marrow samples, e.g. through aspiration.

Bone marrow cells may be obtained from a source of bone marrow, e.g., iliac crests, tibiae, femora, spine, or other bone cavities. Conveniently bone marrow may be aspirated from the bone in accordance with conventional techniques. Other sources of the stem cells include blood, including adult peripheral blood and umbilical cord blood.

The cells are preferably of mammalian origin, i.e. have been isolated from a mammalian sample or are derived from cells isolated from such a sample. Particularly preferred mammals are humans and mice. Further preferred mammals include cows, horses and companion animals.

Various techniques may be employed to separate the cells by initially removing cells of dedicated lineage. Monoclonal antibodies are particularly useful for identifying markers (surface membrane proteins) associated with particular cell lineages and/or stages of differentiation. The antibodies may be attached to a solid support to allow for crude separation. The separation techniques employed should maximize the retention of viability of the fraction to be collected. For "relatively crude" separations, that is, separations where up to 10%, usually not more than about 5%, preferably not more than about 1%, of the total cells present do not have the marker but may remain with the cell population to be retained, since various techniques of different efficacy may be employed. The particular technique employed will depend upon efficiency of separation, cytotoxicity of the methodology, ease and speed of performance, and necessity for sophisticated equipment and/or technical skill.

Procedures for separation may include magnetic separation, using antibody-coated magnetic beads, affinity chromatography, cytotoxic agents joined to a monoclonal antibody or used in conjunction with a monoclonal antibody, e.g., complement and cytotoxins, and "panning" with antibody attached to a solid matrix, e.g., plate, or other convenient technique. Techniques providing accurate separation include fluorescence activated cell sorters, which can have varying degrees of sophistication, e.g., a plurality of color channels, low angle and obtuse light scattering detecting channels, impedance channels, etc.

Conveniently, the antibodies may be conjugated with markers, such as magnetic beads, which allow for direct separation, biotin, which can be removed with avidin or streptavidin bound to a support, fluorochromes, which can be used with a fluorescence activated cell sorter, or the like, to allow for ease of separation of the particular cell type. Any technique may be employed which is not unduly detrimental to the viability of the remaining cells.

Preferably the mononuclear fraction of the blood or bone marrow sample is separated using a Lymphoprep^{™} (Axis Shield) density gradient. CD34⁺ cells can be separated from the mononuclear fraction using MiniMACS (Miltenyi Biotec) technology.

The stem cells can be further characterised by the methods used to obtain them, thus the cells are obtained by a combined affinity purification and selection by adherence method. More particularly, the cells can be labelled with CD34 monoclonal antibody (MAb) and then with (para)magnetic beads which themselves bind to the CD34 MAb. Alternatively, beads which are themselves labelled with the CD34 MAb may be used which bind to the cells. The labelled or bound cells can then be applied to a column and held in place by a magnet; unlabelled cells will be eluted and labelled cells released by removing the magnet (or by removing the column from the magnet).

The thus released CD34⁺ cells can then be incubated at a suitable temperature of e.g. between 35-38° C, preferably 37° C in tissue culture plastic vessels for at least 2 hours, preferably at least 3 hours e.g. 3-5 hours, with non-adherent cells removed by washing with HBSS (Hanks balanced salt solution). The adherent CD34⁺ cells are the stem cells which are used in the methods of the present invention and comprise less than 1% of the total CD34⁺ population. They are preferably a substantially homogenous population, generally uncontaminated by other stem cell subpopulations. Typically less than 30%, preferably less than 20% more preferably less than 5%, most preferably less than 3% of the cells collected are other than the stem cells. However, the Examples show that all individual markers may not be found on every cell in the isolated population and the term "homogenous population" should be interpreted with this in mind. The stem cell population is preferably homogeneous with respect to CD34 expression, adherence to tissue-culture grade plastic and small lymphocyte-like morphology. 'Adherent' cells are defined as those which are able to resist vigorous washing three times without detaching from a solid support (in particular tissue-culture grade plastic or glass). The advantageous properties of the adherent subset of CD34⁺ cells are surprising as the adherent cells would usually be discarded when preparing cells for culture.

The stem cells are capable of self-regeneration, i.e., in accordance with standard definitions of stem cells, stem cells are capable of division to form further stem cells, as well as differentiation to a wide variety of different cell types.

The stem cells are further characterised as CD34⁺, i.e. expressing the antigen CD34, a glycoprotein marker found, but not exclusively so, on stem cells, in particular the stem cells manufactured in the bone marrow (HSC and MSC). The stem cell population may also be enriched for cells expressing the Thy-1 marker, i.e. comprise a significant proportion of cells which are Thy-1⁺. The stem cell population may thus be enriched for Thy-1 relative to the starting cell population (the sample). Example 5 indicates that on average 28.1%, but up to 90% of cells are They-1⁺.

More particularly, the cells may be characterised as CD34⁺, CD38⁻, CD33⁻ and HLA-DR⁻. Preferably the cell population is also enriched for AC133⁺, Thy-1⁺ and/or c-met⁺, more preferably cells are predominantly AC133⁺, Thy-1⁺ and/or c-met⁺.

The stem cells are lymphocyte-like in that they are round mononuclear cells and rather small with a high nucleus: cytoplasm ratio. Such a morphology is associated with primitive stem cells.

They are characterised by an ability to produce differentiated cells in less than 16, e.g. 12-14 days in culture. Preferably differentiation is observed in less than 14 days, e.g. less than 10 days, more preferably in less than 7 days, even 4-5 days.

A sample of the stem cells was deposited with European Collection of Cell Cultures (ECACC), Health Protection Agency, Porton Down, Salisbury, SP4 OJG, UK on 24 September 2004 under accession number 04092401. The deposit was made by the inventor and the cell line was given the name "Stem Cell Omnicyte".

The stem cells may be from any animal, e.g. laboratory, livestock or companion animal; preferably primate and most preferably from humans.

Also disclosed is a culture comprising:
(i) an isolated adult stem cell population wherein said stem cells are CD34⁺, capable of self regeneration and capable of differentiation into ectodermal, mesodermal and endodermal, cells; and
(ii) a medium capable of supporting the growth of said stem cells;
preferably,
(i) a(n isolated) stem cell population wherein said stem cells are CD34⁺, capable of self regeneration and capable of differentiation into ectodermal, mesodermal and endodermal cells and capable of adhering to tissue-culture grade plastic; and
(ii) a medium capable of supporting the growth of said stem cells.

Once stem cells have been isolated, they may be propagated by growing in conditioned medium from stromal cells, such as stromal cells that can be obtained from bone marrow, fetal thymus or fetal liver, and are shown to provide for the secretion of growth factors associated with stem cell maintenance, coculturing with such stromal cells, or in medium comprising maintenance factors supporting the proliferation of stem cells, where the stromal cells may be autologous, allogeneic or xenogeneic. Before using in the coculture, the mixed stromal cell preparations may be freed of haemopoietic cells employing appropriate monoclonal antibodies for removal of the undesired cells, e.g., with antibody-toxin conjugates, antibody and complement, etc. Alternatively, cloned stromal cell lines may be used where the stromal lines may be allogeneic or xenogeneic. Thus, reference above to "medium" includes cells such as stromal cells.

The stem cells and differentiated cells derived therefrom can survive cryopreservation in liquid nitrogen.

Disclosed is a method of isolating an adult stem cell population wherein said stem cells are CD34⁺, capable of self regeneration and capable of differentiation into ectodermal, mesodermal and endodermal cells, which method comprises taking a sample of blood or bone marrow from a subject and extracting said cell population therefrom. Disclosed is a method of isolating a stem cell population wherein said stem cells are CD34⁺, capable of self regeneration and capable of differentiation into ectodermal, mesodermal and endodermal cells and capable of adhering to tissue-culture grade plastic which method comprises taking a sample of blood or bone marrow from a subject and extracting said cell population therefrom. Preferred extraction steps are discussed above and in the case of blood sampling there will typically be a first step of stem cell mobilisation which is preferably performed by administering G-CSF to the subject.

Adhesion to tissue culture plastic is a property of several cell types including marrow mesenchymal stem cells, monocytes and macrophages, but has not previously been used to characterise or isolate a subpopulation of CD34-positive cells. Adherence to tissue culture plastic has been found to be a simple, reproducible and practicable means of selecting primitive stem cells without resort to multiple antibody labelling procedures or other manipulation. The CD34+ cells disclosed herein are also capable of adhering to glass, and glass or other suitable solid supports may thus be used instead of the tissue-culture grade plastic in the methods of the present invention.

The stem cells have utility in research contexts, for example in detecting and evaluating growth factors relevant to stem-cell regeneration. The stem cells may also be of direct utility in the treatment of genetic diseases through gene modification or replacement in autologous stem cells. In particular the cells may be used in the treatment of diseases associated with haemopoietic cells, such as β-thalassemia and sickle cell anemia, where a wild-type gene is introduced into the stem cells. Thus in a further aspect the invention provides a population of stem cells which are CD34⁺, capable of self regeneration, capable of differentiation into ectodermal, mesodermal and endodermal cells and capable of adhering to tissue-culture grade plastic and which are obtained by a method which comprises
(i) subjecting haemopoietic tissue to density gradient separation;
(ii) exposing low density cells to an affinity ligand for CD34;
(iii) recovering cells attached to said CD34 ligand;
(iv) exposing the CD34+ subpopulation to a solid support selected from tissue culture grade plastic and glass;
(v) recovering CD34⁺ cells adherent to said solid support; wherein the genome of the stem cells has been altered by insertion of a region of nucleic acid. In a further aspect, the invention provides said stem cell population for use in therapy. The nucleic acid is preferably a therapeutic gene.

Suitable therapeutic genes will include a wild-type version of a gene which is defective in the patient or a drug resistance gene.

Without additional therapeutic genes the stem cells still have therapeutic utility, e.g. in regenerating the haematopoietic system of a patient deficient in stem cells. Thus, the invention also provides an isolated population of stem cells which are CD34⁺, capable of self regeneration, capable of differentiation into ectodermal, mesodermal and endodermal cells and capable of adhering to tissue-culture grade plastic and which are obtained by a method which comprises
(i) subjecting haemopoietic tissue to density gradient separation;
(ii) exposing low density cells to an affinity ligand for CD34;
(iii) recovering cells attached to said CD34 ligand;
(iv) exposing the CD34+ subpopulation to a solid support selected from tissue culture grade plastic and glass;
(v) recovering CD34⁺ cells adherent to said solid support; for use in therapy.

Further utilities of great interest relate to the generation of different differentiated cell types from the stem cells disclosed herein. As shown in the Figures hereto, it has been possible to generate in advantageously short timescales mesenchymal, haemopoietic, endothelial, epithelial, tube-forming and dendrite-forming cells. The preparation of haemopoietic and mesenchymal cells being particularly preferred. Cells have been observed after less than 14 days with the appearance of liver, nerve, mesenchymal, endothelial, epithelial and haemopoietic cells.

The stem cells are cultured with a cocktail of different cytokines, depending on the desired cell type. The cocktail will typically comprise G-CSF, GM-CSF, IL-3 and stem cell factor, with HGF and FGF being added to stimulate differentiation of hepatocytes; nicotinamide and LY294002 to stimulate differentiation to pancreatic cells and FGF and dibutyryl cyclic AMP to encourage production of nerve cells. Other growth factors are known to the skilled man to be important in the differentiation of other cell types such as bone, cartilage, skeletal and cardiac muscle, kidney, lung, nerve, skin and endocrine tissue. Preferred cell types which are produced in this way are liver, pancreatic, haemopoietic, neuronal and oligodendrocytic cells.

Thus, in a further aspect, the invention provides a method of producing a population of target endodermal, ectodermal, mesenchymal and/or endothelial cells from a population of stem cells, wherein said stem cells are CD34⁺, capable of self regeneration, capable of differentiation into ectodermal, mesodermal and endodermal cells and capable of adhering to tissue-culture grade plastic which method comprises
(i) subjecting haemopoietic tissue to density gradient separation;
(ii) exposing low density cells to an affinity ligand for CD34 ;
(iii) recovering cells attached to said CD34 ligand;
(iv) exposing the CD34+ subpopulation to a solid support selected from tissue culture grade plastic and glass;
(v) recovering CD34⁺ cells adherent to said solid support; and
(vi) culturing said population of stem cells with a plurality of growth factors which causes differentiation of said stem cell population. As described in the Examples and shown in the Figures, successful differentiation may be shown by visual inspection, flow cytometry, reverse transcriptase polymerase chain reaction (RT-PCR) or immunophenotyping. Cells expressing albumin, α-fetoprotein, α1-antitrypsin and hepatocyte growth factor receptor (HGF receptor - c-met) (properties of hepatocytes), vimentin (skeletal muscle and neuronal cells) and smooth muscle actin (muscle cells), in addition to CD34 have, for example, been confirmed.

The differentiated cells will preferably have characteristics, e.g. morphology, and functions of their naturally occurring counterparts. The differentiated cells may, however, be distinguished from naturally occurring and isolated cells by their homogeneity, in this situation homogeneity is with reference to the position of the cells in the normal cell cycle. These differentiated cell populations of the invention will be substantially homogeneous, i.e. all members will largely be at the same point in the cell cycle; in contrast naturally occurring cell populations will be heterologous in this respect.

The cells may be administered in a localised manner, e.g. injected directly into a target organ such as the liver. Alternatively, the cells may be administered at a site remote from the target site, e.g. by intravenous delivery. Tissue targeting may be achieved by forming a complex between the generated cell types and a targeting ligand, such as monoclonal antibodies, cell adhesion molecules and their ligands, cytokine, chemokine and toll-like receptors and their ligands. Such 'complexes' include cells which express the targeting ligand on their cell surface.

The stem cells disclosed herein may be used directly in therapeutic methods, including methods of regeneration and repair, differentiation of the cells may occur in vivo. With stem cells damaged organs may be repaired and/or there may be organ regeneration, also in circumstances where the organ has not been 'damaged' as such but has not developed in the normal way. 'Regeneration' should thus be interpreted broadly to include all methods of organ growth or improvement.

In one preferred embodiment the transplanted cells are adapted to be tracked in vivo, i.e. they incorporate a labelling moiety which means the location of the cells in the body can be identified. Conveniently the cells will incorporate iron compounds e.g. iron oxide, and then MRI can be used to confirm the location of the transplanted cells, in particular to confirm whether they have reached their target tissue. As described in Example 4, the MR agent Resovist® is a suitable iron containing compound which can be taken up by the cells on incubation therewith.

Preferably the patient is human.

The stem cells may also be used in the *in vitro* production of proteins of interest. Thus in a further aspect the invention provides an in vitro method of protein production which comprises culturing the stem cells disclosed herein and then harvesting the cells and recovering one or more of the proteins expressed by said cells.

Animal cells have become the predominant protein expression system for *in vitro* production of target proteins, particularly therapeutic agents, because of their ability to perform post-translational modification (e.g. glycosylation) of proteins. The stem cells disclosed herein can be used in the production of most if not all proteins of therapeutic interest, such as erythropoietin, growth factors, protein hormones such as insulin etc. or synthetic proteins. The cells may be genetically modified in order to provide or enhance production of a particular target protein. However it is a particularly desirable feature of the cell types enabled by the present invention that differentiation to an appropriate cell type (e.g. parenchymal cells) can be performed such that the cells naturally produce the target protein without the need for genetic engineering.

The above described uses of the stem cells disclosed herein are also applicable to non-protein products such as steroids, in both cases, suitable culturing and harvesting techniques are known to the skilled man.

The stem cells have the necessary cellular machinery to propagate vectors such as adenovirus, retrovirus, adeno-associated virus etc.; this is an essential step for current good manufacturing practice (cGMP) preparation of such vectors.

The invention will be further described in the following non-limiting Examples and with reference to the Figures in which:

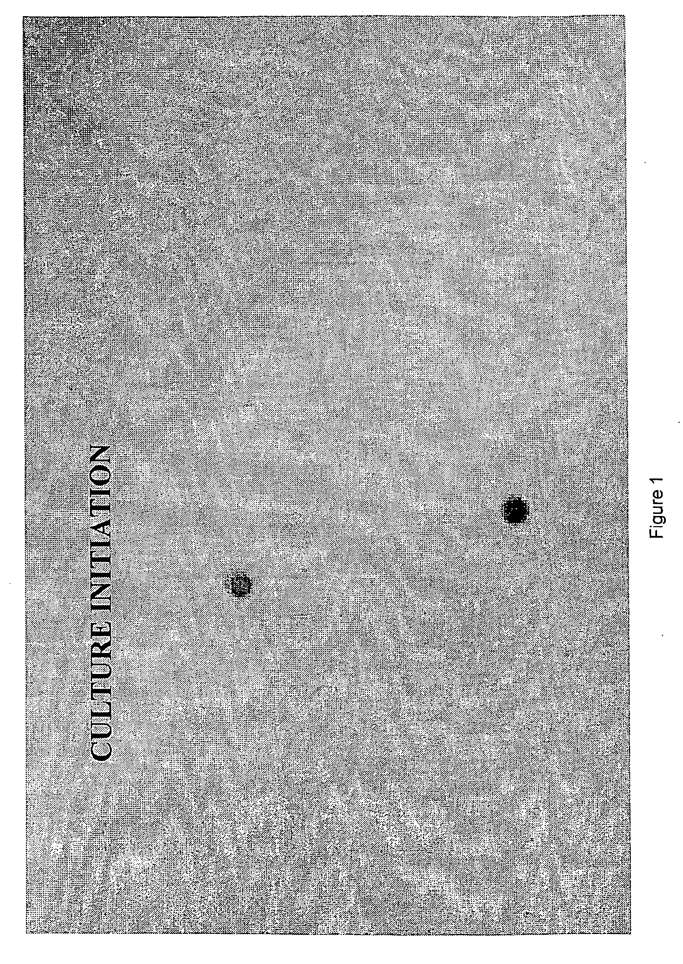

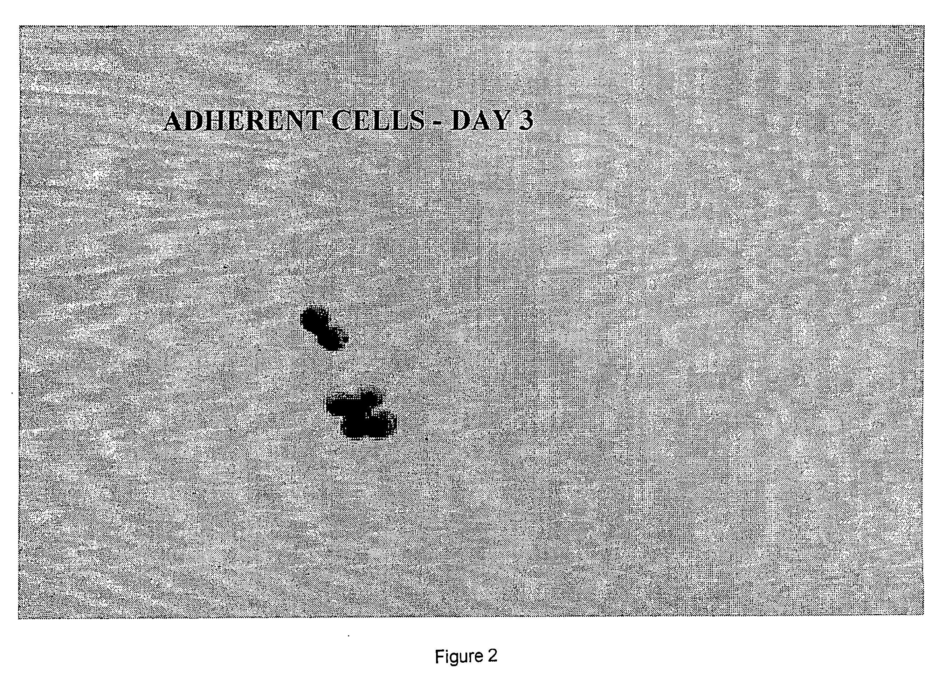

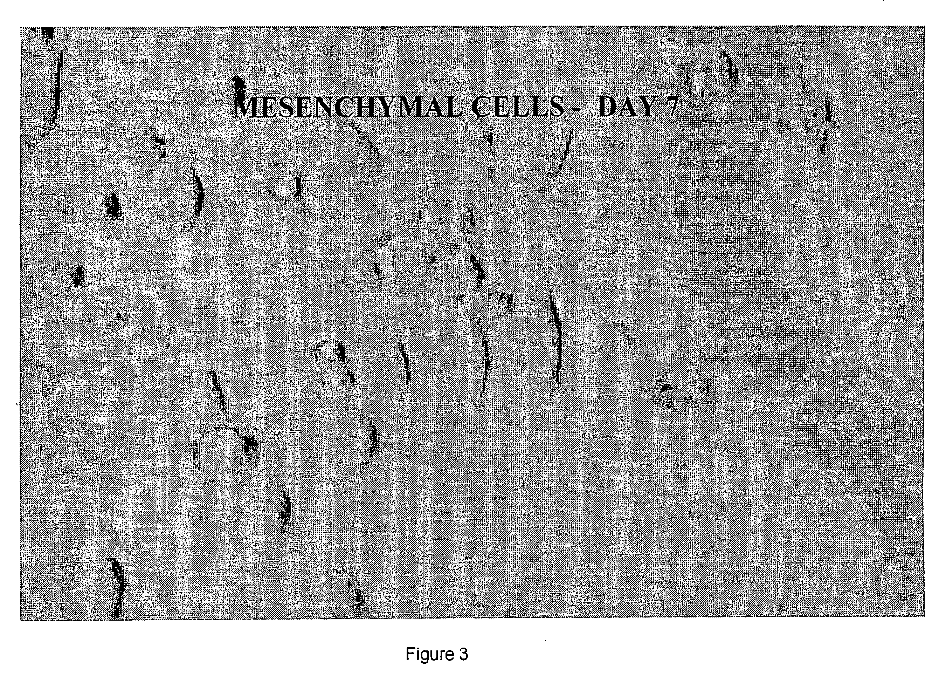

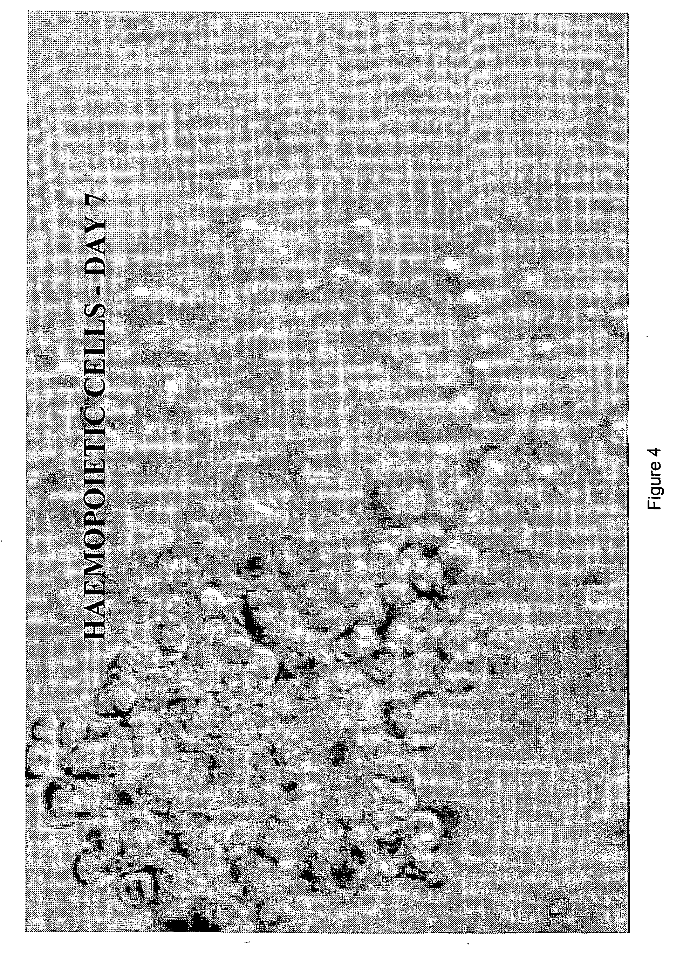

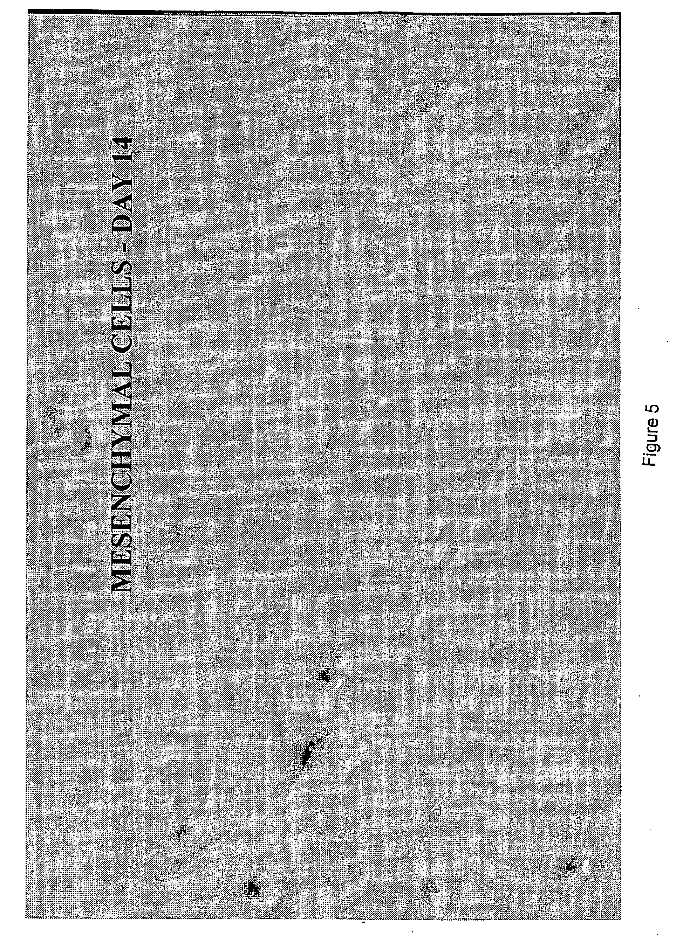

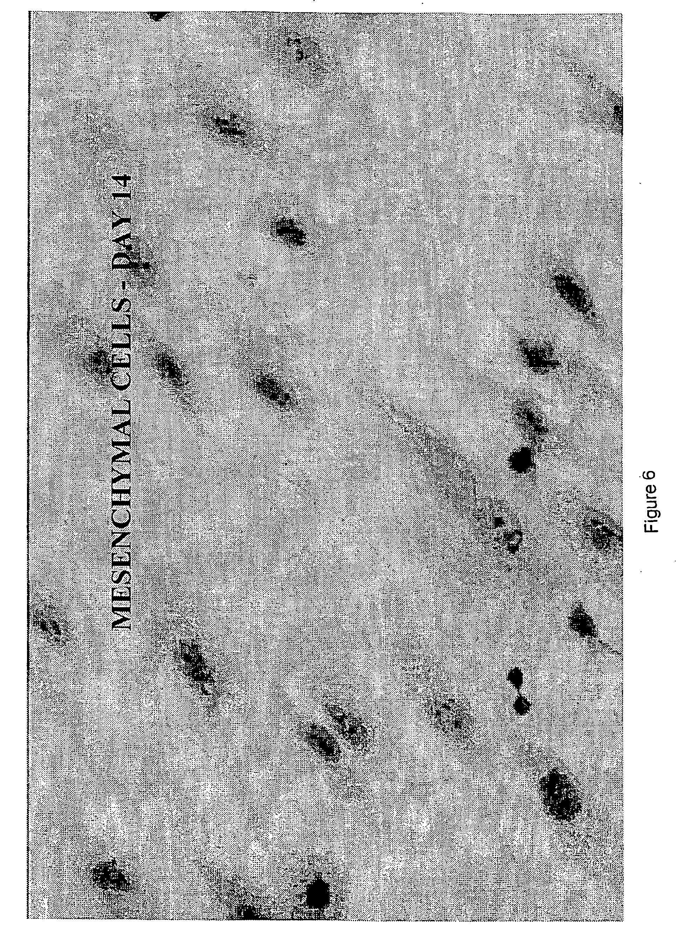

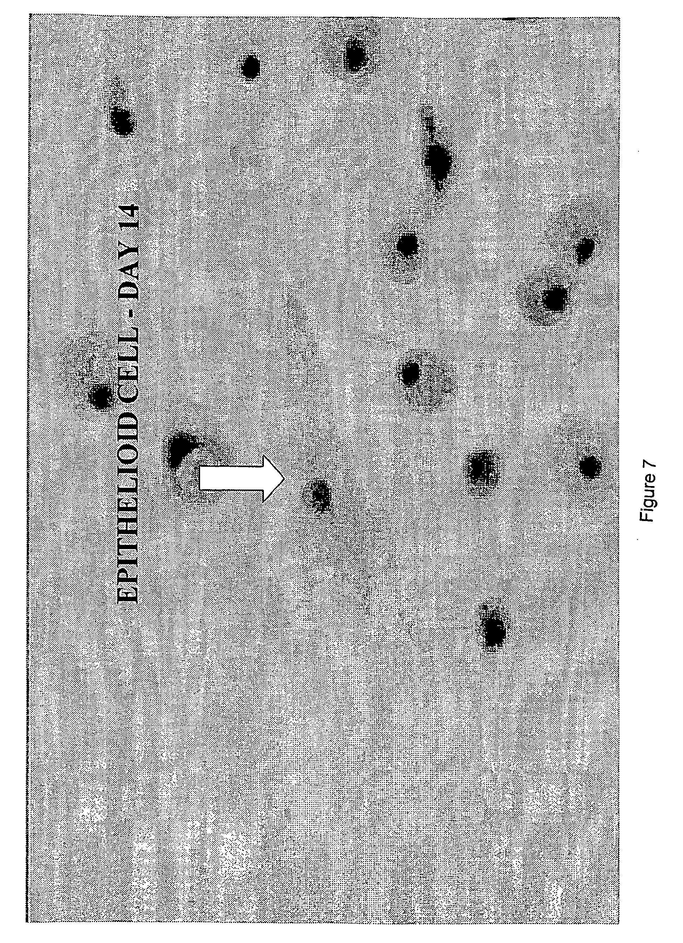

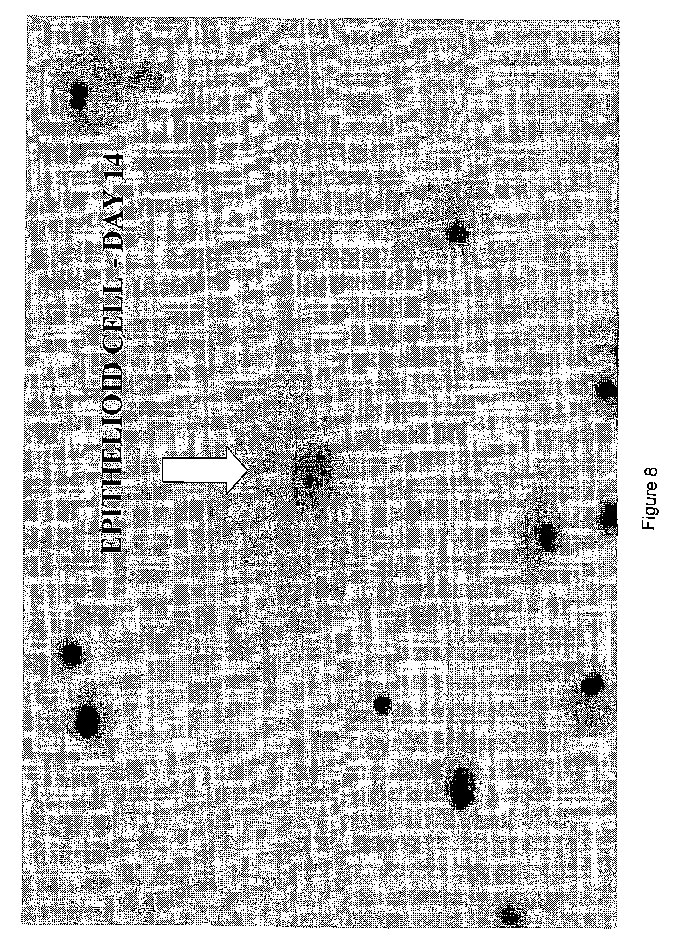

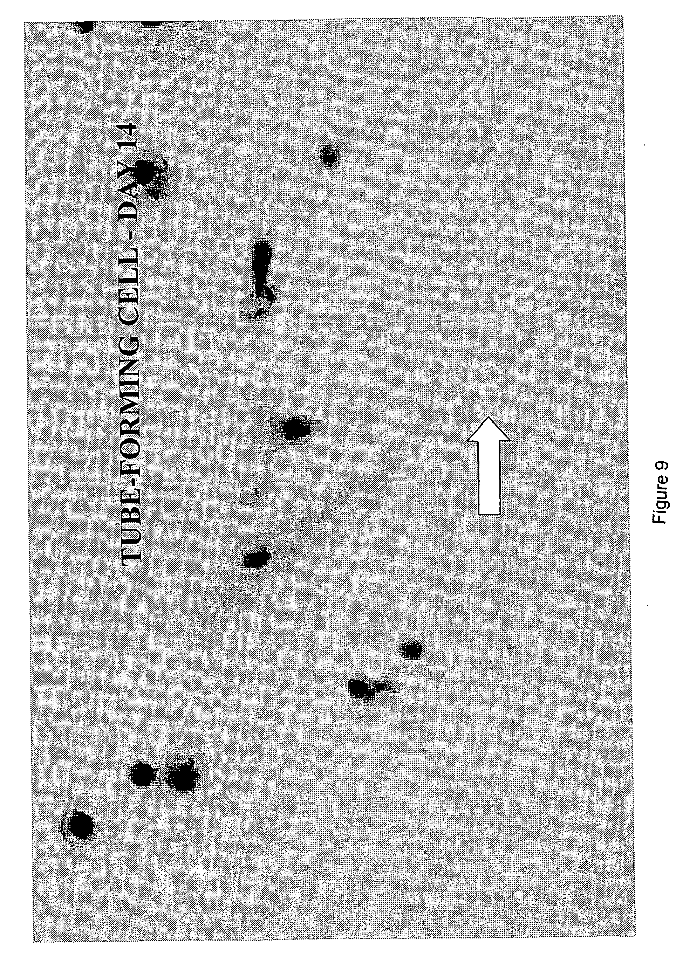

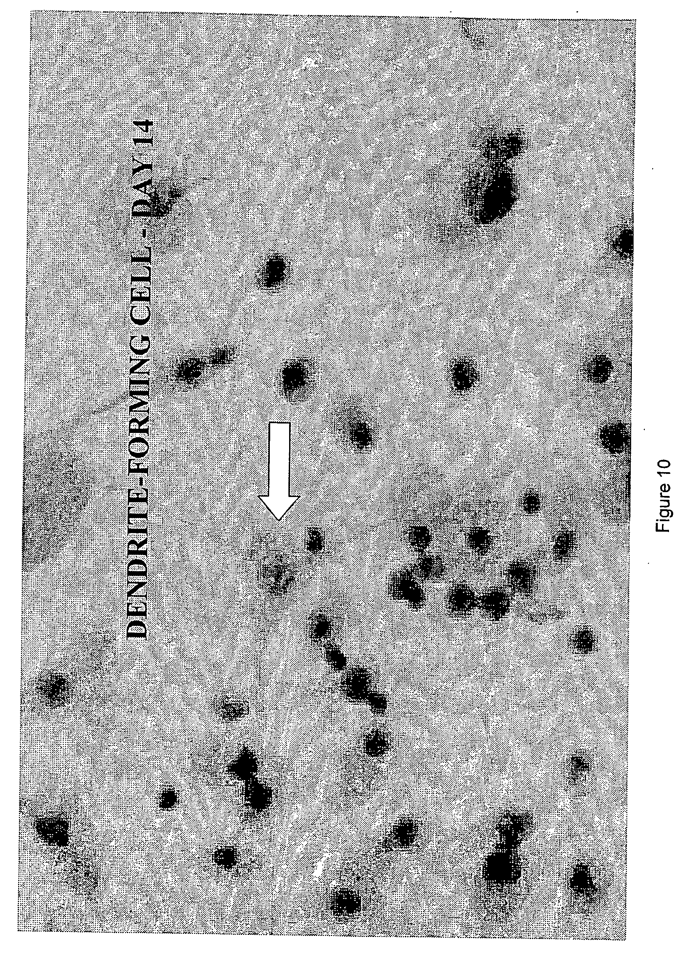

Figures 1-10 are photographs showing the stem cells and their differentiation over time into mesenchymal cells (Fig. 3, 5 and 6), haemopoietic cells (Fig. 4), epithelioid cells (Figs. 7 and 8), tube-forming cells (Fig. 9) and dendrite-forming cells (Fig. 10).
Figure 11 is a photograph showing how CD34⁺ cells were able to take up Resovist® (Schering AG) according to the protocol described in Example 4. According to this figure the individual spots represent the following:
   1. 10⁶ cells, 0.25 mmol Resovist, overnight incubation
   2. 10⁶ cells, 0.25 mmol Resovist + beads, overnight incubation
   3. 10⁶ cells, beads, overnight incubation
   4. 10⁶ cells, negative control (no staining)
   5. 5x10⁵ cells, negative control (no staining)
   6. 5x10⁵ cells, 0.25 mmol Resovist, overnight incubation
   7. 5x10⁵ cells, 0.25 mmol Resovist + beads, overnight incubation
   8. 5x10⁵ cells, beads, overnight incubation
   9. 10⁶ cells, 0.25 mmol Resovist, 2h incubation
   10. 10⁶ cells, 0.25 mmol Resovist + beads, 2h incubation
   11. 10⁶ cells, beads, 2h incubation
   12. 5x10⁵ cells, 0.25 mmol Resovist, 2h
   13. incubation 5x10⁵ cells, 0.25 mmol Resovist + beads, 2h incubation
   14. 5x10⁵ cells, beads, 2h incubation
   15. beads only, no cells.

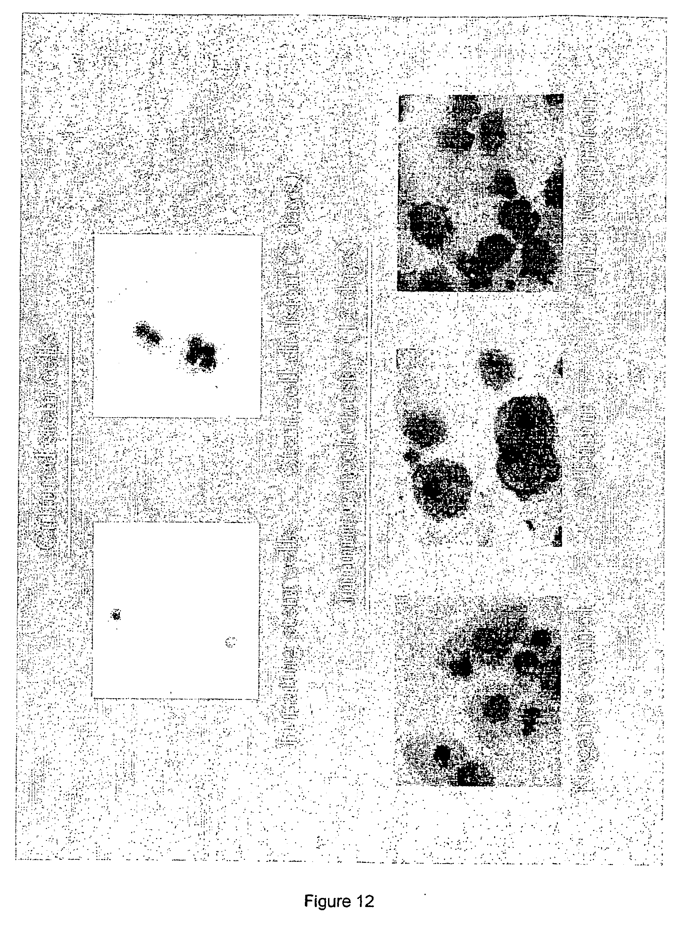

Figure 12 are photographs showing the differentiation of stem cells into liver cells, as evidenced by the presence of the liver cell markers albumin and alpha fetoprotein.

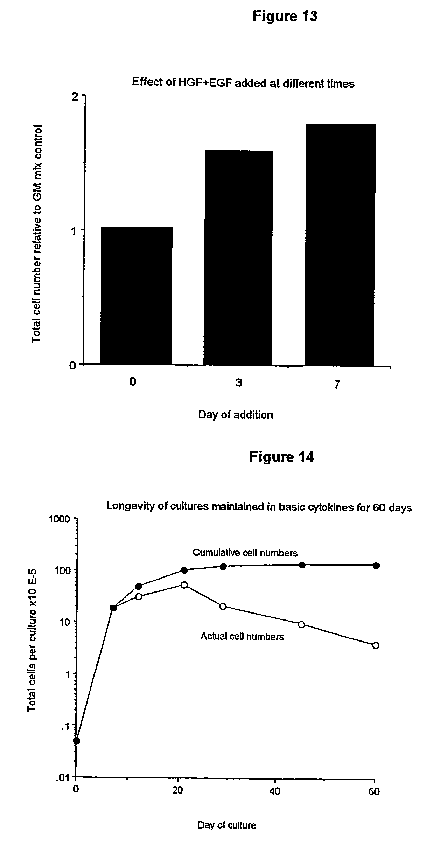

Figure 13 is a graph showing that adding hepatocyte growth factor (HGF) and epidermal growth factor (EGF) to basic cytokines (GM-mix) (GM-mix/basic cytokines are combination of G-CSF, GM-CSF, IL-3 and stem cell factor) on day 7 of culture incubation has a greater impact on cell number than adding them on day 0 or day 3.
Figure 14 is a graph showing the actual and cumulative cell numbers in cultures maintained in basic cytokines for 60 days.

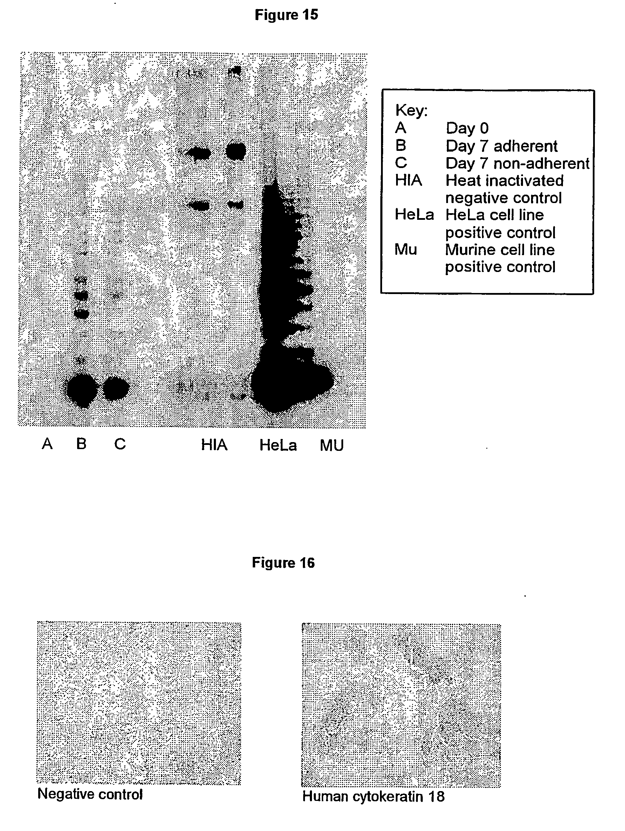

Figure 15 is an autoradiograph of a gel demonstrating telomerase activity of cells after 7 days of culture.
Figure 16 is photographs of microscope slide preparations developed by immunoperoxidase immunocytochemistry showing absence of human cytokeratin 18 from livers of control mice but cytokeratin 18 positivity in livers from transplanted mice.

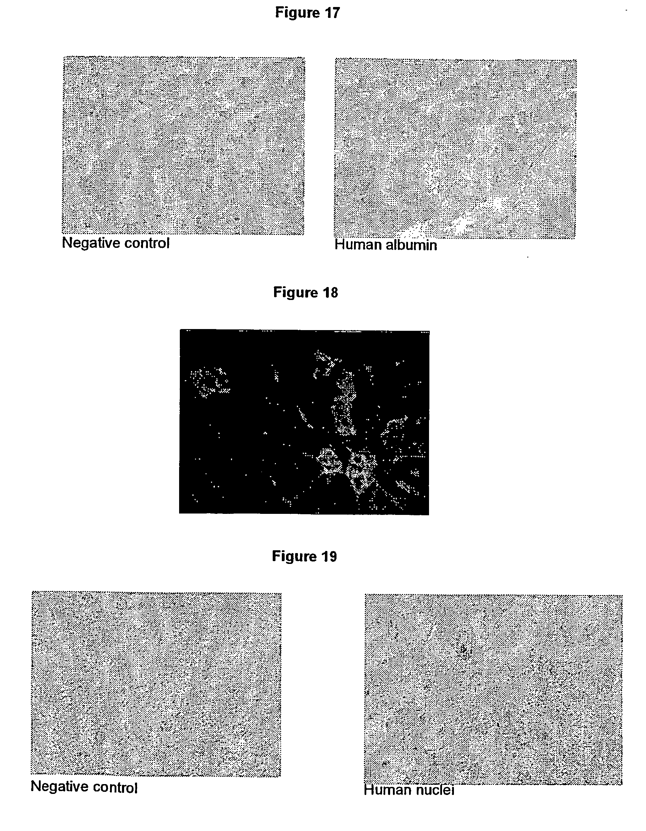

Figure 17 is photographs of microscope slide preparations developed by immunoperoxidase immunocytochemistry showing absence of human albumin from livers of control mice but albumin positivity in livers from transplanted mice.
Figure 18 is a photograph of a three colour immunofluorescent image showing dual staining of cytokeratin 18 and albumin.
Figure 19 is photographs of liver sections from control and transplanted mice showing absence and presence, respectively, of cells stained with an antibody against human nuclei.

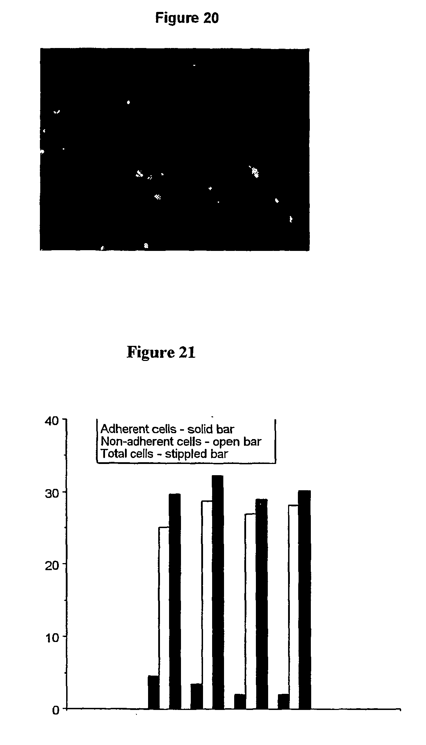

Figure 20 is a photograph showing fluorescent in situ hybridisation analysis of human chromosomes in liver from transplanted mice.
Figure 21 is a graph showing the effects of cryopreserving freshly isolated ASC34, in different serum concentrations, on their subsequent growth in culture

### Examples

### Section A

### Example 1. Cell extraction

Haematopoietic cells were obtained from bone marrow or mobilised blood from normal individuals for transplantation purposes. The mononuclear fraction was separated from the whole using a Lymphoprep^{™} density gradient. CD34⁺ cells were separated from mononuclear fraction using MiniMACS technology. Cells were first labelled with CD34 monoclonal antibody and then with paramagnetic beads. Labelled cells were loaded onto a column held on a magnet, unlabelled cells were eluted and labelled cells released by removing the column from the magnet. The CD34⁺ cells were incubated at 37°C in tissue culture plastic vessels for at least 2h. Non adherent cells were removed by washing with HBSS.

### Example 2. Cell culture

Cells (2 x 10⁵/ml) were incubated in Methylcellulose medium containing serum, 100ng/ml granulocyte colony stimulating factor (G-CSF), 5ng/ml interleukin-3 (IL-3) 20ng/ml stem cell factor (SCF) and 1ng/ml granulocyte macrophage colony stimulation factor (GM-CSF). This cytokine combination may also be referred to as "basic cytokines or "GM-mix". This resulted in heterogeneous populations of cells, which subsequently can be characterised. Selected individual populations were then targeted for differentiation using tailored cytokine cocktails.

### Example 3. Flow cytometry and immunocytochemistry

Flow cytometry. The adherent population that had developed in the cultures was removed by scraping the dish. Cells were fixed in 4% paraformaldehyde and permeabilised. Cells were labelled with monoclonal antibodies conjugated with FITC and analysed using Becton Dickinson flow cytometer.

Immunocytochemistry. The adherent population that had developed in the cultures was removed by scraping the dish. Cells were cytospun onto glass slides and fixed by methanol. Cells were labelled with monoclonal antibodies and visualised using the APAAP (alkaline phosphatase anti-alkaline phosphatase) reaction.

Results of flow cytometry and immunocytochemistry are shown in Table 1 below.

**Table 1.**

| **Antigen** | **Flow cytometry** | **Immunocytochemistry** |
|---|---|---|
| **Albumin** | 9.2%* | Large cells +ve** |
| **Alpha feto protein** | 12.3% | Large cells +ve |
| **Alpha 1 antitripsin** | 20.1% | N/A |
| **cMET (HGF receptor)** | 34.9% | +ve |
| **Smooth muscle actin** | 61.2% | Large cells +ve |
| **GFAP(astrocytes)** | 57.4% | - ve |
| **cKIT** | 22.1% | -ve |
| **Vimentin** | 6.3% | Large cells +ve |
| **hTERT** | 20.1% | - ve |

| | | |
|---|---|---|
| * Denotes % of positive cells in the population ** Denotes that the large cells in the population were positive and the small cells were negative. | | |

Albumin, alpha feto protein, alpha 1 antitrypsin and c-MET (HGF receptor)-are liver cell markers; GFAP (astrocytes) is a brain cell marker; and smooth muscle actin is a marker for mesenchyme cells.

Figure 12 shows cells exhibiting liver cell markers after culturing for 12 days.

### Example 4. Incorporation of iron oxide into cells and labelling cells with paramagnetic beads

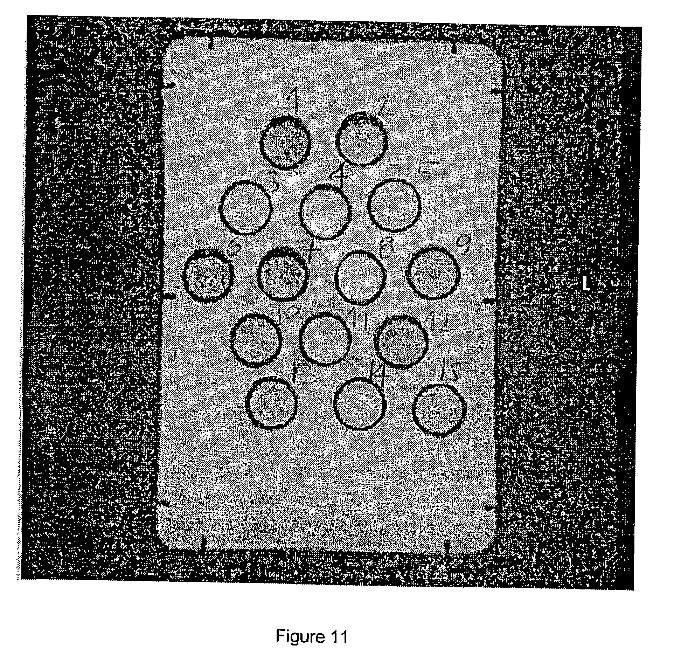

CD34⁺ (10⁶ and 5x10⁵) cells were incubated with 0.25 mmol Resovist® (the brand name of Ferrixan, carboxy-dextran coated iron oxide nanoparticles available from Schering AG) for 2 and 24 hours at 37°C and analysed by MRI. In both cases, positive signal was obtained by MRI suggesting possible use of Resovist in detecting CD34⁺ cells *in vivo.* The results of Figure 11 indicate that the particles can be taken up by the cells and therefore used in tracking the CD34⁺ cells or their differentiated progeny as they move around the body or locate in target tissues.

The in vitro toxicity of Resovist was also tested by Trypan blue exclusion assay and proved to be non-significant (<4%).

The results of this experiment are shown in Fig. 11.

### Section B (Additional examples)

### Example 5: Sources of ASC 34

Bone marrow (BM) obtained from healthy donors by aspiration, mobilised peripheral blood (PB) obtained by leukapheresis from healthy donors who had been given a course of granulocyte colony-stimulating factor (G-CSF) and umbilical cord blood (UCB) obtained from normal full-term deliveries are commonly used sources of ASC 34. Informed consent and Research Ethics Committee approval is required in all cases.

As well as being found in adult haemopietic tissue and cord blood, ASC-34 activity has been detected in adult bone marrow, full-term umbilical cord blood, fetal liver (gestational age 11.6-13.8 weeks) and fetal bone marrow (12.7-15.4 weeks). These cells are distinct from embryonic stem cells because an embryo is considered to become a foetus at 8 weeks post fertilisation.

### Example 6: Purification of a homogeneous population of adherent CD34-positive human stem cells (ASC34)

Initial purification of stem cells to homogeneity is desirable for the subsequent investigation of their potential for self-renewal, differentiation and in vivo engraftment. Such a population is obtained by sequential density gradient separation, immunomagnetic bead selection and differential adherence, and is homogeneous with respect to CD34 expression, adherence to tissue-culture grade plastic or glass and small lymphocyte-like morphology.

The mononuclear cell (MNC) fraction was separated from the whole sample by density gradient centrifugation through Lymphoprep and the CD34-positive cell fraction was then separated from the MNC using MiniMACS technology (Miltenyi Biotech). For this, cells were first labelled with anti-CD34 monoclonal antibody and then with paramagnetic microbeads. The labelled cells were loaded onto a column held on a magnet, the unlabelled cells were eluted and then the labelled cells were released by removing the column from the magnet. The purified (>98%) CD34-positive cells were diluted to 2X10⁵/ml in alpha medium supplemented with 15% serum.

Adherent CD34-positive stem cells (ASC34) were obtained by incubating the CD34-positive cell suspension in tissue culture plastic vessels for at least 2 hours at 37° C.

The non-adherent CD34-positive cells were removed by washing the tissue culture vessels in Hanks' Balanced Salt Solution (HBSS).

Adherent CD34-positive stem cells comprise ∼1% of the total CD34-positive cell population irrespective of the haemopoietic tissue (BM, PB, UCB) used to initiate the cultures. Undifferentiated CD34+ adherent stem cells (ASC34) exhibit a homogeneous small lymphocyte-like morphology with a high nuclear:cytoplasmic ratio. At culture initiation they are widely spaced as single cells on the tissue culture surface. The initiating cells are, by definition, CD34-positive. Antibody-depletion with anti-Thy-1 monoclonal antibody removed virtually all the activity of the adherent CD34+ cells as measured using the production of myeloid colony-forming cells as a readout; according to immunocytochemistry results using cells isolated from 6 independent samples, 28.1% on average, but up to 90% expressed Thy-1. They also express AC133 and c-met localised in the nucleus but not CD3 or CD19. They do not express CD33, CD38 or HLA-DR. They are non-cycling cells that are resistant to treatment with the cell cycle active drug, 5-fluorouracil.

Table 2 demonstrates that the ASC34 are significantly more homogeneous than nonadherent CD34-positive cells, with respect to expression of CD33, CD38 and HLA-DR.

**Table 2: Percent antigen negative CD34-positive cells in adherent and non-adherent fractions**

| Antigen | Adherent CD34+ | Non-adherent CD34+ | P value ** |
|---|---|---|---|
| CD38 (n=4) | 87.6±3.1* | 17.4±15.8 | 0.008 |
| CD33 (n=4) | 87.5±4.9 | 58.8±17.2 | 0.02 |
| HLA-DR (n=4) | 71.4+8.9 | 43.3±16.9 | 0.03 |

| | | | |
|---|---|---|---|
| *mean ± sem (standard error of the mean); ** Mann-Whitney U test comparing adherent and non-adherent CD34+ cells; n=number of samples tested. | | | |

### Example 7: Use of adherence to tissue culture plastic as a selectable marker

Adhesion to tissue culture plastic is a property of several cell types including marrow mesenchymal stem cells, monocytes and macrophages, but has not previously been used to characterise a subpopulation of CD34-positive cells. Adherence to tissue culture plastic has been found to be a simple, reproducible and practicable means of selecting primitive stem cells without resort to multiple antibody labelling procedures or other manipulation. Another advantage is that the cells utilise the plastic as their initial growth substrate and do not require transfer to a separate culture environment after purification.

CD34-positive cells suspended in culture medium are introduced into tissue culture plastic vessels at a concentration of 5x10⁵ cells per ml. The vessels are incubated at 37°C in humidified 5% CO₂ in air. Non-adherent CD34-positive cells, comprising 99% of the total Cd34-positive population, are removed by thorough washing with culture medium. ASC34 bind readily to glass but not to non-tissue culture grade plastics.

ASC34 can be retrieved for further study or manipulation by mechanically removing them from the tissue culture plastic using a cell scraper. Trypsin and accutase are ineffective.

### Example 8: Use of mobilised blood as a source of adherent CD34-positive human stem cells (ASC34)

The number of cells available to start a culture is one limiting factor in the progress of tissue regeneration from cultured stem cells. Bone marrow and umbilical cord blood are favoured sources. However, PBPC (Peripheral Blood Progenitor Cell) harvests yield many more cells to start a culture thus reducing the degree of amplification and time required to generate a clinically useful product.

Donors (autologous or allogeneic) are treated with a one-week course of G-CSF at 5mg/kg administered subcutaneously. Cells are harvested by leukapheresis using a programmed apheresis machine. Typical yields of cells range from 5-10x10¹⁰, most of which are mononuclear cells and ∼1% (5-10x10⁸) are CD34+. The CD34-positive cells are separated using a CliniMacs sytem (scaled up version of the MiniMacs). By direct observation, the ASC34 are ∼1% of the CD34-positive population (5-10x106). This estimate has been confirmed by limiting dilution analysis of ASC34 activity using haemopoietic colony-forming cell assays as the readout. Thus, a 3-4 log expansion which is achievable over a period of 1-2 weeks, would yield 5-100x10⁹ cells for clinical applications.

### Example 9: Culture of ASC34

Phase 1 conditions: The first phase of the culture consists of expanding the adherent cells. The ASC34 are overlaid with methylcellulose containing serum (Methocult H4230; Metachem Diagnostics, Northampton, UK) and a basic cocktail of cytokines (100 ng/ml G-CSF (Chugai Pharma, London, UK)1 ng/ml GM-CSF, 5ng/ml IL-3, 20 ng/ml SCF (all from First Link, West Midlands, UK)). The cultures are incubated at 37°C in 5% humidified CO₂ in air. The ASC34 divide and self-renew to form colonies of adherent stem cells and then adherent cells that exhibit morphologies characteristic of mesenchymal, epithelial, vascular and neural cell types. A 40-fold increase in adherent cell number is achieved in the first week of culture. In addition, non-adherent cells are released into the methylcellulose where large colonies of haemopoietic cells (leukocytes) are found.

Omission of methyl cellulose from the culture mileu reduced cell production by 60%. Increasing CD34+ cell numbers seeded beyond a routine 5x10⁵/ml did not lead to a commensurate improvement in cell production. Cell numbers were not improved by the addition of 4mM lithium chloride.

Phase 2 conditions: In phase 2, cells are transferred to liquid culture conditions with the addition of further cytokines to induce selective cell differentiation as required. Suitable cytokines for differently directed differentiations are listed below.

**Table 3: Suitable conditions for directed differentiation**

| Tissues | Examples of additives |
|---|---|
| Nerve | DMSO; butylated hydroxyanisole; isobutyl methylxanthine; b-FGF; FGF-8 |
| Pancreas | bFGF; EGF; activin A; betacellin; HGF; nicotinamide |
| Skeletal muscle | EGF; PDGF; 5-azacytidine |
| Heart | Norepinephrine; forskolin; retinoic acid |
| Liver | EGF; HGF; FGF-4 |
| Bone | BMP-4 |
| Cartilage | TGF-beta; dexamethosone; BMP-6 |

As can be seen in Figure 13, some cytokines were more effective when they were added on day 7 of culture than when they were added on day 0 or day 3. Table 4 shows the effects of different cytokines and combinations on total cell number in 2 week-old cultures.

**Table 4: Effects of different cytokines and cytokine combinations on cell yield*.**

| Cytokine(s) added | Day 0 | Day 7 |
|---|---|---|
| aFGF | 2.5 | 0.98 |
| bFGF | 1.2 | 1.0 |
| FGF4 | 1.0 | 0.99 |
| IGF | 1.75 | 0.62 |
| VEGF | 1.0 | 1.01 |
| HGF | 1.4 | 0.8 |
| bFGF + IGF | 1.38 | 1.18 |
| VEGF + IGF | 0.87 | 0.71 |
| HGF + FGF4 | 1.2 | - |
| HGF + VEGF | 1.14 | 1.14 |
| HGF + EGF | 1.0 | 1.8 |
| HGF + β cellulin | - | 1.0 |
| HGF+EGF+ β | - | 3.5 |
| cellulin+activin A KGF + nicotinamide + glucose | - | 0.83 |

| | | |
|---|---|---|
| * cell yield relative to GM mix only ie ratio between cell yield in prescence of added cytokines:cell yield in presence of GM-mix alone | | |

The data show that additional cytokines had no major effects on cell number overall. More importantly, combinations designed to induce differentiation (HGF+EGF+ β cellulin+activin A/ KGF + nicotinamide + glucose) did not reduce the cell yield.

Cultures can be maintained in phase 2 conditions for 60 days (Figure 14)

### Example 10: Telomerase activity

Telomerase activity was measured using the TRAP assay. Cells were transferred into lx CHAPS buffer and the resulting lysate assayed for protein concentration using a DC assay (Bio-Rad), normalised to 77ng/µl and diluted 1:10, 1:40 and 1:160 with CHAPS buffer. Lysates were analysed using the TRAPeze telomerase detection kit (Intergen). The TS primer was labelled at 37°C for 20 min and heat denatured at 85°C for 5 min. The PCR was then run for 28 cycles with an annealing temperature of 59°C. The resulting TRAP products were diluted in TRAP loading dye and run on a 12.5% acrylamide-0.5 x TBE gel, dried and exposed to X-ray film.

At the initiation of the culture the cells did not express telomerase activity as assessed by the TRAP assay, which is consistent with the quiescent nature of the cells at isolation. Significant telomerase activity is evident in cells from 7-day old cultures (Figure 15) which is consistent with cell proliferation at that stage.

### Example 11: Polymerase chain reaction

Lysates were prepared from ASC34 (day 0) and from ASC34-derived cells after 7 and 14 days of culture in basic cytokines or with the addition of HGF or EGF as indicated in the key. In further experiments, cells were analysed for up to 35 days in culture. Separate lysates were prepared from the adherent and non-adherent cell fractions of the cultured cells. Gene expression was analysed by PCR.

Gene expression by ASC 34 at day 0

### Self-renewal and pluripotency markers.

| | |
|---|---|
| Rex-1 | redox-sensing transcriptional repressor |
| Oct 4 | octamer-binding transcription factor-4 |
| Nanog | homeodomain protein promoting ES cell self-renewal |

### Haemopoietic cell markers

| | |
|---|---|
| CD34 | haemopoietic stem cell marker |
| CD133 | cholesterol-binding protein prominin 1. Lipid raft marker |
| RECAM | platelet-endothelial adhesion molecule |
| VWF | von Willibrand factor. Coagulation |
| TAL-1 | T cell acute leukaemia-1. Basic helix-loop-helix protein |
| CXCR4 | chemokine receptor for SDF-1. Important for stem cell homing and engraftment |
| Angiopoietin 1 | Ligand for Tie 2. Maintains haemopoietic stem cell quiescence |
| Tie 2 | Receptor for angiopoietin-1. Maintains haemopoietic stem cell quiescence. |

### Skeletal muscle markers

| | |
|---|---|
| TNNT1 | skeletal slow troponin 1 |
| Desmin | major intermediate filament protein of muscle |
| Nebulin | structural component of striated muscle sarcomere filaments |

### Heart

| | |
|---|---|
| Connexin-43 | gap junction component in cardiomyocytes |
| GATA-4 | zinc finger transcription factor binding to FOG2 in cardiomyocytes |

### Nerve

| | |
|---|---|
| CXCR4 | chemokine receptor for SDF-1 on neural precursors |
| Connexin-43 | gap junction component on astrocytes |

### Endothelium

| | |
|---|---|
| CD34 | haemopoietic stem cell marker. Also expressed by endothelial cells |
| CD133 | cholesterol binding protein prominin 1. Lipid raft marker |
| VEGF | vascular endothelial growth factor |
| KDR | kinase insert domain receptor. A receptor for VEGF |
| Angiopoietin 1 | angiogenesis factor. Ligand for Tie 2 |
| Angiopoietin 2 | angiogeneis factor. Ligand for Tek |
| Tie 2 | receptor for angiopoietin 1 |
| CXCR4 | chemokine receptor for SDF-1. Important for vascularisation |
| PECAM | platelet-endothelial cell adhesion molecule |
| ICAM 2 | intercellular adhesion molecule 2. Mediates leukocyte extravasation |
| VE cadherin | formation of adherens junctions |
| TAL-1 | T cell acute leukaemia-1. |
| VWF | von Willibrand factor. Coagulation factor |

### Liver

| | |
|---|---|
| Alpha-1 antitrypsin | |
| Cytokeratin 18 | |
| Nestin | |
| Vimentin | |
| c-met CD34 | receptor for hepatocyte growth factor haemopoietic stem cell antigen. Also expressed on candidate liver stem cells. |

### Pancreas

| | |
|---|---|
| NGN-3 | target of pdx-1 in beta cell differentiation |

Gene expression by ASC 34 generated cells after 14 days in culture.

### Haemopoietic cell markers

| | |
|---|---|
| CD133 | cholesterol-binding protein prominin 1. Lipidraft marker |
| PECAM | platelet-endothelial cell adhesion molecule |
| VWF | von Willibrand factor. Coagulation |
| TAL-1 | T cell acute leukaemia-1. Basic helix-loop-helix protein |
| CXCR4 | chemokine receptor for SDF-1. Important for stem cell homing and engraftment |
| Angiopoietin 1 | ligand for Tie 2. Maintains haemopoietic stem cell quiescence |

### Skeletal muscle markers

| | |
|---|---|
| Nebulin | giant cytoskeletal protein. Structural component of striated muscle sarcomere filaments |
| Heart | |
| Troponin | 1subunit of troponin complex |
| Nebulin | giant cytoskeletal protein |
| Nerve | |
| CXCR4 | chemokine receptor for SDF-1 on neural precursors |
| Endothelium | |
| CD133 | cholesterol-binding protein prominin 1. Lipid raft marker |
| VEGF | vascular endothelial growth factor |
| Angiopoietin 1 | angiogenesis factor. Ligand for Tie 2 |
| Angiopoietin 2 | angiogenesis factor. Ligand for Tek |
| CXCR4 | chemokine receptor for SDF-1. Important for vascularisation |
| PECAM | platelet-endothelial cell adhesion molecule |
| ICAM 2 | intercellular adhesion molecule 2. Mediates leukocyte extravasation |
| VWF | von Willibrand factor. Coagulation |
| TAL-1 | T cell acute leukaemia-1. Basic helix-loop-helix protein |
| Nebulin | giant cytoskeletal protein |
| Liver | |
| Alpha-1 | antitrypsin protease inhibitor |
| Cytokeratin 18 | cytoskeletal component |
| LDLR | low density lipoprotein receptor. Role in cholesterol homeostasis |
| Albumin | carrier protein for steroids, fatty acids and thyroid hormones |
| HGF | hepatocyte growth factor |
| HNF3-B | hepatocyte nuclear factor 3 beta. Transcription factor |
| Transferrin | iron transport |
| AFP | alphafeto protein |
| Pancreas | |
| Pax-6 | |
| Pdx-1 | |
| Insulin | counteracts hyperglycemia and stimulates lipogenesis |
| IGF-1 | insulin-like growth factor 1. Somatomedin |
| HNF3-B | hepatocyte nuclear factor 3 beta. Transcription factor expressed in glucagon-producing islet cells. |
| NeuroD-1 | regulates insulin gene expression |
| NGN3 | |

Expression of insulin, PDX-1, Neuro D-1 and NGN3 gene expression by cells cultured in basic cytokines (GM-mix) was investigated at weekly intervals. The results show that insulin was expressed from day 7 to day 35, PDX-1 from day 7 to day 35, NeuroD-1 from day 14 to day 35 and NGN3 from day 21 to day 35. Thus, expression of genes involved in insulin production was sustained for 3-4 weeks with the most comprehensive expression occurring in cultures 3-5 weeks old. Thus in a preferred embodiment of the present invention the stem cells are able to generate progeny which express genes involved in insulin production (insulin, PDX-1, Neuro D-1 and NGN3).

### Example 12: ASC34 differentiation into haemopoietic cells

Cells harvested from the cultures after 14 days were plated into standard haemopoietic colony-forming cell assays. Typically, ^{∼}10³ granulocyte-macrophage colonies formed, representing ^{∼}1% of the total cells in the culture. Erythroid BFU-e, megakaryocytic (Mk-CFC) and multipotential (GEMM) colony-forming cells were also evident when the ASC34-derived cells were harvested and grown in haemopoietic colony assays. More importantly, ASC34 give rise to bone marrow stroma-adherent stem/progenitor cells that form blast cell colonies/"cobblestone" areas when inoculated onto preformed cultured stroma and are capable of long term haemopoietic cell production on prolonged in vitro incubation (5 weeks).

Cytospin preparations of the cells were made and stained with Romanowsky cytochemical stains. This revealed morphological evidence of granulocytic, monocyte-macrophage, mekakaryocytic and erythroid cell differentiation.

### Example 13: Engraftment in animal models

Experiment 1: Intravenous or subcutaneous injection of large numbers (1 x 10⁶) of ASC34-derived cells into nude mice did not cause any mortality or discernible morbidity.

Experiment 2: Nude mice that had been treated with a liver toxin (1g/kg Thioacetamide) received 1x10⁶ ASC34-derived cells including candidate hepatocytes by injection directly into the spleen. Nude mouse recipients of toxin only served as controls. All of the control mice died by day 7 whilst all of the recipients of cells survived, irrespective of whether or not they had been treated with cyclosporin (CsA) (Table 5)

**Table 5: Results of transplanting ASC34-derived cells into mice with liver damage**

| | Tx* only control n=4 | Tx+cells n=6 | Tx+cells+cyclosporin n=6 |
|---|---|---|---|
| % mortality at day 7 | 100 | 0 | 0 |

| | | | |
|---|---|---|---|
| * liver toxin | | | |

### Experiment 3

Three experimental groups were set up: group 1, TA + cells; group 2, TA + CsA + cells; group 3, TA only. Doses of CsA were administered to group 2 animals twice per week. All mice in group 3 died within 2 days after treatment with TA. Animals in groups 1 and 2 survived until sacrificed for tissue sampling (days 1, 8 and 15). Liver sections from group 2 mice stained positive for human cytokeratin 18, a liver-specific marker demonstrating the presence of human ASC34-derived cells. No staining was seen on sections from control animals (Figure 16).

### Experiment 4

The anti-Fas antibody JO2 was used to induce an ongoing chronic form of liver failure. Each animal received 250 µg JO2/kg/week for 4 weeks and 1x10⁶ cells intrasplenically 24 hours after the first JO2 injection. CsA was given twice weekly. Of 12 animals, there were 2 procedure-related deaths after 2 days and one animal survived for 82 days. The remainder were sacrificed for examination (one on day 2, two on day 8, three on day 13 and three on day 21).

The analyses presented in Figures 16-20 demonstrate that human cells engrafted in the livers of the mice and produced albumin and cytokeratin 18.

### Example 14: Cryopreservation and storage

Cells (recovered adherent CD34-positive fraction or their progeny generated in culture) were suspended in 30% serum/10% DMSO in cryopreservation vials and placed in an alcohol freezing bath at -80°C overnight. The frozen vials were then transferred to the vapour phase of liquid nitrogen for storage. Cells were recovered from frozen by rapidly thawing the vials in a 37°C water bath, diluting the contents with medium and washing the cells.

Prior to processing, the leukapheresis product can be stored for 24 hours at 37°C without any deleterious effect on subsequent viability or growth in vitro (data not shown). When the adherent cells were purified before cryopreservation in 10% DMSO plus 30-50% serum then thawed they retained 92±4.8% (mean±SD) viability and there was no effect on their growth in vitro (Figure 21).

These results have practical implications
1. *Prior to processing*: Leukapheresis product can be stored for 24 hours at 4°C before processing. Therefore, chilled leukaphereses can be transported worldwide from the collection centre to the processing centre.
2. *After purification*: ASC34 can be suspended in 10%DMSO/30-50% serum and frozen in liquid nitrogen. On thawing they retain 90-100% viability, re-adhere to tissue culture plastic and proliferate in culture like fresh cells. Thus, isolated functional ASC34 can be transported worldwide or stored long-term.
3. After culture: Cells cultured for 14 days have been cryopreserved, thawed and recultured. Cells cultured for 14 days can be held at ambient temperature and retain viability. Thus, cultured cells can be transported worldwide or stored long-term.

### Example 15: Summary of differences between ASC-34 and mesenchymal stem cells (MSC)

Mesenchymal stem cells are a separate population of cells that can be derived from adult bone marrow. MSC (also called Multipotent Adult Progenitor Cells - MAPC) and ASC-34 cells (disclosed herein) exhibit important differences that are summarised below. It should also be noted that MSC/MAPC cells do not express CD34.

| Characteristic | Multipotent adult progenitor cell (MAPC) * | ASC-34 |
|---|---|---|
| Known phenotype in vivo | -¹ | + |
| Prospective isolation from tissue | - | + |
| Known ontogeny | - | + |
| Homogeneity | - | + |
| Quantitative assay | - | + |
| Quiescent in vivo | ? | +² |
| Produce haemopoietic cells in culture | - | + |

| | | |
|---|---|---|
| * Ref: Javazon, Beggs and Flake. Exp Hematol 2004, 32:414. ¹ - MAPC are only found after prolonged culture. They have not been identified in fresh bone marrow samples. ²- ASC-34 cells are resistant to 5 fluorouracil | | |

## Claims

1. A method of producing a population of target endodermal, ectodermal, mesenchymal and/or endothelial cells from a population of stem cells, wherein said stem cells are CD34⁺, capable of self regeneration, capable of differentiation into ectodermal, mesodermal and endodermal cells and capable of adhering to tissue-culture grade plastic
which method comprises
(i) subjecting haemopoietic tissue to density gradient separation;
(ii) exposing low density cells to an affinity ligand for CD34;
(iii) recovering cells attached to said CD34 ligand;
(iv) exposing the CD34+ subpopulation to a solid support selected from tissue culture grade plastic and glass;
(v) recovering CD34⁺ cells adherent to said solid support; and
(vi) culturing said population of stem cells with a plurality of growth factors which causes differentiation of said stem cell population.

2. A method according to claim 1, wherein the target cell is selected from the group comprising pancreatic, liver, nerve, epithelial, glandular epithelial, bone, skeletal muscle, cardiac muscle, kidney, lung, skin and endocrine cells.

3. A method according to claim 2, wherein the target cell is a hepatocyte, a neuronal cell or an oligodendrocytic cell.

4. The method according to any one of claims 1 to 3, wherein said target endodermal cells express genes involved in insulin production.

5. The method according to any one of claims 1 to 4, wherein said stem cells are further **characterised by** their ability to adhere to tissue-culture grade plastic within 3 hours after isolation, and to remain adherent for at least 72 hours.

6. The method according to any one of claims 1 to 5, wherein said stem cells are CD33⁻, CD38⁻, HLA-DR⁻, CD3⁻ and CD19⁻.

7. The method according to any one of claims 1-6, wherein said stem cell population is enriched for cells which are also Thy-1⁺.

8. The method according to any one of claims 1-7, wherein said stem cell population is enriched for cells which are also AC133⁺ and/or c-met⁺.

9. The method according to any one of claims 1-8, wherein said stem cell population expresses genes encoding Rex-1, Oct 4, Nanog, CD34, CD133, PECAM, VWF, Tal-1, CXCR4, Angiopoietin 1, Tie 2, TNNT1, Desmin, Nebulin, Connexin-43, GATA-4, VEGF, KDR, Angiopoietin 2, ICAM-2, VE cadherin, Alpha-1-antitrypsin, Cytokeratin 18, Nestin, Vimentin and c-met.

10. The method according to any one of claims 1-9 wherein the stem cells are adult stem cells.

11. The method according to any one of claims 1-10 wherein the stem cell population comprises fetal cells obtained from a non-fetal sample such as an umbilical cord sample.

12. The method according to any one of claims 1-11 wherein the stem cells have accession No. ECACC 04092401.

13. The method according to any one of claims 1-12 wherein said stem cell population is mammalian in origin.

14. The method according to any one of claims 1-13 wherein said stem cell population is human in origin.

15. The method according to any one of claims 1-14 wherein said stem cell population is murine, equine or bovine in origin or is isolated or derived from a sample taken from a companion animal.

16. The method according to any one of claims 1-15 wherein said stem cell population does not require feeder layers during culturing thereof.

17. A population of stem cells which are CD34⁺, capable of self regeneration, capable of differentiation into ectodermal, mesodermal and endodermal cells and capable of adhering to tissue-culture grade plastic
and which are obtained by a method which comprises
(i) subjecting haemopoietic tissue to density gradient separation;
(ii) exposing low density cells to an affinity ligand for CD34;
(iii) recovering cells attached to said CD34 ligand;
(iv) exposing the CD34+ subpopulation to a solid support selected from tissue culture grade plastic and glass;
(v) recovering CD34⁺ cells adherent to said solid support; wherein the genome of the stem cells has been altered by insertion of a region of nucleic acid.

18. The cell population of claim 17, wherein the genome is altered by insertion of DNA using a DNA virus, RNA virus or a retroviral vector.

19. A population of stem cells which are CD34⁺, capable of self regeneration, capable of differentiation into ectodermal, mesodermal and endodermal cells and capable of adhering to tissue-culture grade plastic
and which are obtained by a method which comprises
(i) subjecting haemopoietic tissue to density gradient separation;
(ii) exposing low density cells to an affinity ligand for CD34;
(iii) recovering cells attached to said CD34 ligand;
(iv) exposing the CD34+ subpopulation to a solid support selected from tissue culture grade plastic and glass;
(v) recovering CD34⁺ cells adherent to said solid support wherein a portion of the genome has been inactivated, e.g. through the presence of an antisense nucleic acid molecule, a ribozyme sequence or an inhibitory RNA sequence.

20. An isolated population of stem cells which are CD34⁺, capable of self regeneration, capable of differentiation into ectodermal, mesodermal and endodermal cells and capable of adhering to tissue-culture grade plastic
and which are obtained by a method which comprises
(i) subjecting haemopoietic tissue to density gradient separation;
(ii) exposing low density cells to an affinity ligand for CD34;
(iii) recovering cells attached to said CD34 ligand;
(iv) exposing the CD34+ subpopulation to a solid support selected from tissue culture grade plastic and glass;
(v) recovering CD34⁺ cells adherent to said solid support; for use in therapy.

21. An isolated population of stem cells according to any one of claims 17 to 19 for use in therapy.

22. An isolated stem cell population for use according to claim 20 or 21 in regenerating an organ or repairing a damaged organ.

23. An isolated stem cell population for use according to claim 22, wherein the organ is selected from the group comprising the immune system, liver, lung, pancreas, bone, cartilage, muscle, skin, brain or nervous system and heart or circulatory system.

24. An isolated stem cell population for use according to any one of claims 20-23, wherein the cells are labelled with a traceable marker, preferably iron oxide or paramagnetic beads.

25. Use of
(a) an isolated population of stem cells which are CD34⁺, capable of self regeneration, capable of differentiation into ectodermal, mesodermal and endodermal cells and capable of adhering to tissue-culture grade plastic
and obtained by a method which comprises
(i) subjecting haemopoietic tissue to density gradient separation;
(ii) exposing low density cells to an affinity ligand for CD34;
(iii) recovering cells attached to said CD34 ligand;
(iv) exposing the CD34+ subpopulation to a solid support selected from tissue culture grade plastic and glass;
(v) recovering CD34⁺ cells adherent to said solid support ;or
(b) an isolated population of stem cells according to any one of claims 17 to 19 in the manufacture of a medicament for regenerating an organ or repairing a damaged organ.

26. An *in vitro* method of protein production which comprises
(a) culturing a population of stem cells which are CD34⁺, capable of self regeneration, capable of differentiation into ectodermal, mesodermal and endodermal cells and capable of adhering to tissue-culture grade plastic
and which are obtained by a method which comprises
(i) subjecting haemopoietic tissue to density gradient separation;
(ii) exposing low density cells to an affinity ligand for CD34;
(iii) recovering cells attached to said CD34 ligand;
(iv) exposing the CD34+ subpopulation to a solid support selected from tissue culture grade plastic and glass;
(v) recovering CD34⁺ cells adherent to said solid support and
(b) recovering one or more of the proteins expressed by said cells.

27. An *in vitro* method of protein production according to claim 26, wherein said protein is insulin or albumin.

## Patentansprüche

1. Verfahren zur Herstellung einer Population von Endoderm-, Ektoderm-, Mesenchym- und/oder Endothel-zielzellen aus einer Population von Stammzellen, wobei es sich bei den Stammzellen um CD34⁺-Zellen mit der Fähigkeit zur Selbstregenerierung, zur Differenzierung zu Ektoderm-, Mesoderm- und Endodermzellen sowie zum Haften an Gewebekulturqualität-Kunststoff handelt,
wobei man bei dem Verfahren (i) hämatopoetisches Gewebe einer Dichtegradiententrennung unterzieht;
(ii) Zellen niedriger Dichte mit einem Affinitätsliganden für CD34 in Kontakt bringt;
(iii) an dem CD34-Liganden gebundene Zellen gewinnt;
(iv) die CD34'-Subpopulation mit einem aus Gewebekulturqualität-Kunststoff und -Glas ausgewählten festen Träger in Kontakt bringt;
(v) an dem festen Träger haftende CD34⁺-Zellen gewinnt; und
(vi) die Population von Stammzellen mit mehreren Wachstumsfaktoren kultiviert, was zur Differenzierung der Stammzellpopulation führt.

2. Verfahren nach Anspruch 1, wobei die zielzelle aus der Bauchspeicheldrüsen-, Leber-, Nerven-, Epithel-, Drüsenepithel-, Knochen-, Skelettmuskel-, Herzmuskel-, Nieren-, Lungen-, Haut- und endokrine Zellen umfassenden Gruppe ausgewählt ist.

3. Verfahren nach Anspruch 2, wobei es sich bei der zielzelle um einen Hepatozyten, eine neuronale Zelle oder einen Oligodendrozyten handelt.

4. Verfahren nach einem der Ansprüche 1 bis 3, wobei die Endoderm-Zielzellen an der Insulinproduktion beteiligte Gene exprimieren.

5. Verfahren nach einem der Ansprüche 1 bis 4, wobei die Stammzellen ferner durch ihre Fähigkeit gekennzeichnet sind, innerhalb von 3 Stunden nach der Isolierung an Gewebekulturqualität-Kunststoff zu haften und wenigstens 72 Stunden lang haften zu bleiben.

6. Verfahren nach einem der Ansprüche 1 bis 5, wobei es sich bei den Stammzellen um CD33⁻-, CD38⁻-, HLA-DR⁻-, CD3⁻- und CD19⁻-Zellen handelt.

7. Verfahren nach einem der Ansprüche 1-6, wobei die Stammzellpopulation an Zellen angereichert wird, bei denen es sich auch um Thy-1⁺-Zellen handelt.

8. Verfahren nach einem der Ansprüche 1-7, wobei die Stammzellpopulation an Zellen angereichert wird, bei denen es sich auch um AC133⁺- und/oder c-met⁺-Zellen handelt.

9. Verfahren nach einem der Ansprüche 1-8, wobei die Stammzellpopulation für Rex-1, Oct 4, Nanog, CD34, CD133, PECAM, VWF, Tal-1, CXCR4, Angiopoetin 1, Tie 2, TNNT1, Desmin, Nebulin, Connexin-43, GATA-4, VEGF, KDR, Angiopoetin 2, ICAM-2, VE-Cadherin, Alpha-1-Antitrypsin, Cytokeratin 18, Nestin, Vimentin und c-met codierende Gene exprimiert.

10. Verfahren nach einem der Ansprüche 1-9, wobei es sich bei den Stammzellen um adulte Stammzellen handelt.

11. Verfahren nach einem der Ansprüche 1-10, wobei die Stammzellpopulation einer nichtfötalen Probe, wie etwa einer Nabelschnurprobe, entnommene fötale Zellen umfasst.

12. Verfahren nach einem der Ansprüche 1-11, wobei die Stammzellen die Zugangs-Nr. ECACC 04092401 besitzen.

13. Verfahren nach einem der Ansprüche 1-12, wobei die Stammzellpopulation aus einem Säuger stammt.

14. Verfahren nach einem der Ansprüche 1-13, wobei die Stammzellpopulation aus einem Menschen stammt.

15. Verfahren nach einem der Ansprüche 1-14, wobei die Stammzellpopulation aus einer Maus, einem Pferd oder Rind stammt oder aus einer einem Heimtier entnommenen Probe isoliert oder erhalten wird.

16. Verfahren nach einem der Ansprüche 1-15, wobei die Stammzellpopulation während ihrer Kultivierung keine Feeder-Schichten benötigt.

17. Population von Stammzellen, bei denen es sich um CD34⁺-Zellen mit der Fähigkeit zur Selbstregenerierung, zur Differenzierung zu Ektoderm-, Mesoderm- und Endodermzellen sowie zum Haften an Gewebekulturqualität-Kunststoff handelt und die mit einem Verfahren erhalten werden, bei dem man
(i) hämatopoetisches Gewebe einer Dichtegradiententrennung unterzieht;
(ii) Zellen niedriger Dichte mit einem Affinitätsliganden für CD34 in Kontakt bringt;
(iii) an dem CD34--Liganden gebundene Zellen gewinnt;
(iv) die CD34⁺-Subpopulation mit einem aus Gewebekulturqualität-Kunststoff und -Glas ausgewählten festen Träger in Kontakt bringt;
(v) an dem festen Träger haftende CD34⁺-Zellen gewinnt; wobei das Genom der Stammzellen durch Insertion eines Bereichs von Nukleinsäure verändert wurde.

18. Zellpopulation gemäß Anspruch 17, wobei das Genom durch Insertion von DNA unter Verwendung eines DNA-virus, RNA-Virus oder eines Retrovirus-Vektors verändert ist.

19. Population von Stammzellen, bei denen es sich zum CD34⁺-Zellen mit der Fähigkeit zur Selbstregenerierung, zur Differenzierung zu Ektoderm-, Mesoderm- und Endodermzellen sowie zum Haften an Gewebekulturqualität-Kunststoff handelt und die mit einem Verfahren erhalten werden, bei dem man
(i) hämatopoetisches Gewebe einer Dichtegradiententrennung unterzieht;
(ii) zellen niedriger Dichte mit einem Affinitätsliganden für CD34 in Kontakt bringt;
(iii) an dem CD34-Liganden gebundene zellen gewinnt;
(iv) die CD34⁺-Subpopulation mit einem aus Gewebekulturqualität-Kunststoff und -Glas ausgewählten festen Träger in Kontakt bringt;
(v) an dem festen Träger haftende CD34⁺-Zellen gewinnt, wobei ein Teil des Genoms inaktiviert wurde, beispielsweise durch die Gegenwart eines Antisense-Nukleinsäuremoleküls, einer Ribozym-Sequenz oder einer Inhibitor-RNA-Sequenz.

20. Isolierte Population von Stammzellen, bei denen es sich um CD34⁺-Zellen mit der Fähigkeit zur Selbstregenerierung, zur Differenzierung zu Ektoderm-, Mesoderm- und Endodermzellen sowie zum Haften an Gewebekulturqualität-Kunststoff handelt und die mit einem Verfahren erhalten werden, bei dem man
(i) hämatopoetisches Gewebe einer Dichtegradiententrennung unterzieht;
(ii) Zellen niedriger Dichte mit einem Affinitätsliganden für CD34 in Kontakt bringt;
(iii) an dem CD34-Liganden gebundene Zellen gewinnt;
(iv) die CD34⁺-Subpopulation mit einem aus Gewebekulturqualität-Kunststoff und -Glas ausgewählten festen Träger in Kontakt bringt;
(v) an dem festen Träger haftende CD34⁺-Zellen gewinnt; zur Verwendung in der Therapie.

21. Isolierte Population von Stammzellen nach einem der Ansprüche 17 bis 19 zur Verwendung in der Therapie.

22. Isolierte Stammzellpopulation zur Verwendung nach Anspruch 20 oder 21 bei der Regenerierung eines Organs oder der Reparatur eines beschädigten Organs.

23. Isolierte Stammzellpopulation zur Verwendung nach Anspruch 22, wobei das Organ aus der das Immunsystem, Leber, Lunge, Bauchspeicheldrüse, Knochen, Knorpel, Muskel, Haut, Gehirn oder Nervensystem sowie Herz oder Kreislaufsystem umfassenden Gruppe ausgewählt ist.

24. Isolierte Stammzellpopulation zur Verwendung nach einem der Ansprüche 20-23, wobei die Zellen mit einem auffindbaren Marker, vorzugsweise Eisenoxid oder paramagnetischen Kügelchen, markiert sind.

25. Verwendung
(a) einer isolierten Population von Stammzellen, bei denen es sich um CD34⁺-Zellen mit der Fähigkeit zur Selbstregenerierung, zur Differenzierung zu Ektoderm-, Mesoderm- und Endodermzellen sowie zum Haften an Gewebekulturqualität-Kunststoff handelt und die mit einem Verfahren erhalten werden, bei dem man
(i) hämatopoetisches Gewebe einer Dichtegradiententrennung unterzieht;
(ii) Zellen niedriger Dichte mit einem Affinitätsliganden für CD34 in Kontakt bringt;
(iii) an dem CD34-Liganden gebundene Zellen gewinnt;
(iv) die CD34⁺-Subpopulation mit einem aus Gewebekulturqualität-Kunststoff und -Glas ausgewählten festen Träger in Kontakt bringt;
(v) an dem festen Träger haftende CD34⁺-Zellen gewinnt; oder
(b) einer isolierten Population von Stammzellen nach einem der Ansprüche 17 bis 19 bei der Herstellung eines Arzneimittels zur Regenerierung eines Organs oder Reparatur eines beschädigten Organs.

26. In-vitro-Verfahren zur Proteinherstellung, bei dem man
(a) eine Population von Stammzellen, bei denen es sich um CD34⁺-zellen mit der Fähigkeit zur Selbstregenerierung, zur Differenzierung zu Ektoderm-, Mesoderm- und Endodermzellen sowie zum Haften an Gewebekulturqualität-Kunststoff handelt und die mit einem Verfahren erhalten werden, bei dem man
(i) hämatopoetisches Gewebe einer Dichtegradiententrennung unterzieht;
(ii) Zellen niedriger Dichte mit einem Affinitätsliganden für CD34 in Kontakt bringt;
(iii) an dem CD34-Liganden gebundene zellen gewinnt;
(iv) die CD34⁺-Subpopulation mit einem aus Gewebekulturqualität-Kunststoff und -Glas ausgewählten festen Träger in Kontakt bringt;
(v) an dem festen Träger haftende CD34⁺-Zellen gewinnt, kultiviert und
(b) eines oder mehrere der von den zellen exprimierten Proteine gewinnt.

27. In-vitro-Verfahren zur Proteinherstellung nach Anspruch 26, wobei es sich bei dem Protein um Insulin oder Albumin handelt.

## Revendications

1. Procédé de production d'une population de cellules cibles endodermiques, ectodermiques, mésenchymateuses et/ou endothéliales provenant d'une population de cellules souches, dans laquelle lesdites cellules souches sont de type CD34+, sont capables de s'auto-régénérer, capables de se différencier en cellules ectodermiques, mésodermiques et endodermiques, et capables d'adhérer à du plastique d'une qualité pour culture tissulaire
lequel procédé comprend les étapes consistant à :
(i) soumettre un tissu hématopoïétique à une séparation par gradient de densité ;
(ii) exposer les cellules de faible densité à un ligand d'affinité pour les CD34 ;
(iii) récupérer les cellules fixées au dit ligand de CD34 ;
(iv) exposer la sous-population de CD34+ à un support solide, choisi parmi des plastiques et verres d'une qualité pour culture tissulaire ;
(v) récupérer les cellules CD34⁺ qui ont adhéré au dit support solide ; et
(vi) cultiver ladite population de cellules souches avec une pluralité de facteurs de croissance, qui provoquent une différenciation de ladite population de cellules souches.

2. Procédé selon la revendication 1, dans lequel la cellule cible est choisie dans le groupe comprenant les cellules pancréatiques, hépatiques, nerveuses, épithéliales, épithéliales glandulaires, osseuses, des muscles squelettiques, du muscle cardiaque, rénales, pulmonaires, cutanées et endocrines.

3. Procédé selon la revendication 2, dans lequel la cellule cible est un hépatocyte, une cellule neuronale ou une cellule oligodendrocytique.

4. Procédé selon l'une quelconque des revendications 1 à 3, dans lequel lesdites cellules endodermiques cibles expriment des gènes impliqués dans la production d'insuline.

5. Procédé selon l'une quelconque des revendications 1 à 4, dans lequel lesdites cellules souches sont **caractérisées en outre par** leur faculté d'adhérer à du plastique d'une qualité pour culture tissulaire dans les 3 heures après leur isolement et de rester fixées pendant au moins 72 heures.

6. Procédé selon l'une quelconque des revendications 1 à 5, dans lequel lesdites cellules souches sont des CD33⁻, CD38⁻, HLA-DR⁻, CD3⁻ et CD19⁻.

7. Procédé selon l'une quelconque des revendications 1 à 6, dans lequel ladite population de cellules souches est enrichie pour les cellules qui sont aussi Thy-1⁺.

8. Procédé selon l'une quelconque des revendications 1 à 7, dans lequel ladite population de cellules souches est enrichie pour les cellules qui sont aussi AC133⁺ et/ou c-met⁺.

9. Procédé selon l'une quelconque des revendications 1 à 8, dans lequel ladite population de cellules souches exprime des gènes codant pour Rex-1, Oct 4, Nanog, CD34, CD133, PECAM, VWF, Tal-1, CXCR4, Angiopoïétine 1, Tie 2, TNNT1, Desmine, Nébuline, Connexine-43, GATA-4, VEGF, KDR, Angiopoïétine 2, ICAM-2, cadhérine VE, antitrypsine alpha-1, Cytokératine 18, Nestine, Vimentine et c-met.

10. Procédé selon l'une quelconque des revendications 1 à 9, dans lequel les cellules souches sont des cellules souches adultes.

11. Procédé selon l'une quelconque des revendications 1 à 10, dans lequel la population de cellules souches comprend des cellules foetales obtenues à partir d'un échantillon non foetal, tel qu'un échantillon de cordon ombilical.

12. Procédé selon l'une quelconque des revendications 1 à 11, dans lequel les cellules souches ont le numéro d'ordre ECACC 04092401.

13. Procédé selon l'une quelconque des revendications 1 à 12, dans lequel ladite population de cellules souches est d'origine mammalienne.

14. Procédé selon l'une quelconque des revendications 1 à 13, dans lequel ladite population de cellules souches est d'origine humaine.

15. Procédé selon l'une quelconque des revendications 1 à 14, dans lequel ladite population de cellules souches est d'origine murine, équine ou bovine ou bien est isolée à partir d'un échantillon prélevé sur un animal de compagnie ou est dérivée de celui-ci.

16. Procédé selon l'une quelconque des revendications 1 à 15, dans lequel ladite population de cellules souches ne requiert pas de couches nourricières lors de la culture de celle-ci.

17. Population de cellules souches qui sont de type CD34⁺, sont capables de s'auto-régénérer, capables de se différencier en cellules ectodermiques, mésodermiques et endodermiques, et capables d'adhérer à du plastique d'une qualité pour culture tissulaire et qui sont obtenues par un procédé qui comprend les étapes consistant à
(i) soumettre un tissu hématopoïétique à une séparation par gradient de densité ;
(ii) exposer les cellules de faible densité à un ligand d'affinité pour les CD34 ;
(iii) récupérer les cellules fixées au dit ligand de CD34 ;
(iv) exposer la sous-population de CD34+ à un support solide, choisi parmi des plastiques et verres d'une qualité pour culture tissulaire ;
(v) récupérer les cellules CD34⁺ qui ont adhéré
au dit support solide ; dans lesquelles le génome des cellules souches a été altéré par l'insertion d'une région d'acide nucléique.

18. Population de cellules selon la revendication 17, dans laquelle le génome est altéré par l'insertion d'ADN en utilisant un virus à ADN, un virus à ARN ou un vecteur rétroviral.

19. Population de cellules souches qui sont de type CD34⁺, sont capables de s'auto-régénérer, capables de se différencier en cellules ectodermiques, mésodermiques et endodermiques, et capables d'adhérer à du plastique d'une qualité pour culture tissulaire et qui sont obtenues par un procédé qui comprend les étapes consistant à
(i) soumettre un tissu hématopoïétique à une séparation par gradient de densité ;
(ii) exposer les cellules de faible densité à un ligand d'affinité pour les CD34 ;
(iii) récupérer les cellules fixées au dit ligand de CD34 ;
(iv) exposer la sous-population de CD34+ à un support solide, choisi parmi des plastiques et verres d'une qualité pour culture tissulaire ;
(v) récupérer les cellules CD34⁺ qui ont adhéré
au dit support solide, dans lesquelles une partie du génome a été inactivée, à titre d'exemple au moyen de la présence d'une molécule d'acide nucléique antisens, une séquence de ribozyme ou une séquence d'ARN d'inhibition.

20. Population isolée de cellules souches qui sont de type CD34⁺, sont capables de s'auto-régénérer, capables de se différencier en cellules ectodermiques, mésodermiques et endodermiques, et capables d'adhérer à du plastique d'une qualité pour culture tissulaire et qui sont obtenues par un procédé qui comprend les étapes consistant à
(i) soumettre un tissu hématopoïétique à une séparation par gradient de densité ;
(ii) exposer les cellules de faible densité à un ligand d'affinité pour les CD34 ;
(iii) récupérer les cellules fixées au dit ligand de CD34 ;
(iv) exposer la sous-population de CD34+ à un support solide, choisi parmi des plastiques et verres d'une qualité pour culture tissulaire ;
(v) récupérer les cellules CD34⁺ qui ont adhéré
au dit support solide ; pour être utilisées dans une thérapie.

21. Population isolée de cellules souches, selon l'une quelconque des revendications 17 à 19, destinée à être utilisée dans une thérapie.

22. Population isolée de cellules souches destinée à être utilisée, selon la revendication 20 ou la 21, dans la régénération d'un organe ou la réparation d'un organe endommagé.

23. Population isolée de cellules souches destinée à être utilisée selon la revendication 22, dans laquelle l'organe est choisi dans le groupe comprenant le système immunitaire, les : foie, poumon, pancréas, os, cartilage, muscle, peau, cerveau, ou bien le système nerveux ainsi que le système cardiaque ou circulatoire.

24. Population isolée de cellules souches destinée à être utilisée selon l'une quelconque des revendications 20 à 23, dans laquelle les cellules sont marquées avec un marqueur pouvant être tracé, de préférence des billes d'oxyde de fer ou paramagnétiques.

25. Utilisation
(a) d'une population isolée de cellules souches, qui sont de type CD34+, sont capables de s'auto-régénérer, capables de se différencier en cellules ectodermiques, mésodermiques et endodermiques, et capables d'adhérer à du plastique d'une qualité pour culture tissulaire et obtenues par un procédé qui comprend les étapes consistant à
(i) soumettre un tissu hématopoïétique à une séparation par gradient de densité ;
(ii) exposer les cellules de faible densité à un ligand d'affinité pour les CD34 ;
(iii) récupérer les cellules fixées au dit ligand de CD34 ;
(iv) exposer la sous-population de CD34+ à un support solide, choisi parmi des plastiques et verres d'une qualité pour culture tissulaire ;
(v) récupérer les cellules CD34⁺ qui ont adhéré au dit support solide ; ou
(b) d'une population isolée de cellules souches, selon l'une quelconque des revendications 17 à 19,
dans la fabrication d'un médicament destiné à régénérer un organe ou réparer un organe endommagé.

26. Procédé *in vitro* de production de protéines, qui comprend les étapes consistant à
(a) cultiver une population de cellules souches, qui sont de type CD34+, sont capables de s'auto-régénérer, capables de se différencier en cellules ectodermiques, mésodermiques et endodermiques, et capables d'adhérer à du plastique d'une qualité pour culture tissulaire et qui sont obtenues par un procédé qui comprend les étapes consistant à
(i) soumettre un tissu hématopoïétique à une séparation par gradient de densité ;
(ii) exposer les cellules de faible densité à un ligand d'affinité pour les CD34 ;
(iii) récupérer les cellules fixées au dit ligand de CD34 ;
(iv) exposer la sous-population de CD34+ à un support solide, choisi parmi des plastiques et verres d'une qualité pour culture tissulaire ;
(v) récupérer les cellules CD34⁺ qui ont adhéré au dit support solide ; et
(b) récupérer une ou plusieurs des protéines exprimées par lesdites cellules.

27. Procédé *in vitro* de production d'une protéine, selon la revendication 26, dans lequel ladite protéine est l'insuline ou l'albumine.
